(19) **Europäisches Patentamt**
**European Patent Office**
**Office européen des brevets**

(11) **EP 4 675 632 A1**

(12) **EUROPÄISCHE PATENTANMELDUNG**

(43) Veröffentlichungstag:
07.01.2026 Patentblatt 2026/02

(21) Anmeldenummer: 24186811.6

(22) Anmeldetag: 05.07.2024

(51) Internationale Patentklassifikation (IPC):
G16H 20/40 (2018.01)  G16H 10/60 (2018.01)
A61B 34/00 (2016.01)  G06F 18/00 (2023.01)
G06N 5/00 (2023.01)  G06N 20/00 (2019.01)
G16H 30/40 (2018.01)  G16H 50/70 (2018.01)
G16H 50/30 (2018.01)  G16H 70/00 (2018.01)

(52) Gemeinsame Patentklassifikation (CPC):
G16H 10/60; A61B 34/00; G06F 18/00;
G06N 3/045; G06N 3/08; G06N 5/00; G06N 20/00;
G16H 20/40; G16H 30/40; G16H 50/30;
G16H 50/70; G16H 70/00

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**GE KH MA MD TN**

(71) Anmelder: **Siemens Healthineers AG**
**91301 Forchheim (DE)**

(72) Erfinder: **Birkhold, Annette**
**70193 Stuttgart (DE)**

(74) Vertreter: **Siemens Healthineers**
**Patent Attorneys**
**Postfach 22 16 34**
**80506 München (DE)**

(54) **VERFAHREN, EINRICHTUNGEN, COMPUTERPROGRAMME UND DATENTRÄGER ZUM BEREITSTELLEN EINER TRAINIERTEN UNTERSTÜTZUNGSFUNKTION FÜR EINEN BILDÜBERWACHTEN MEDIZINISCHEN EINGRIFF UND ZUR UNTERSTÜTZUNG BEI EINEM SOLCHEN EINGRIFF SOWIE EINGRIFFSANORDNUNG**

(57) Die Erfindung betrifft ein Verfahren zum Bereitstellen einer trainierten Unterstützungsfunktion (15) für einen bildüberwachten medizinischen Eingriff an einer Eingriffsanordnung (31), die wenigstens eine technische Eingriffseinrichtung (32, 35) aufweist, von der wenigstens eine eine medizinische Bildaufnahmeeinrichtung (33) zur Bildüberwachung des Eingriffs ist, zur Unterstützung einer Bedienperson, umfassend:
- Bereitstellen einer für mehrere Datentypen, umfassend Bilde, Videos und strukturierten Text, vortrainierten generativen Funktion (1), die aus Eingangsdaten (3) der Datentypen auf die Eingangsdaten (3) bezogene Ausgangsdaten (2) generiert, als untrainierte Unterstützungsfunktion,
- Trainieren der Unterstützungsfunktion mit auf den bildüberwachten medizinischen Eingriff bezogenen Trainingsdatensätzen (8), wobei die Trainingsdatensätze (8) für mehrere Zeitpunkte einer Eingriffsdauer jedes in den Trainingsdatensätzen (8) beschriebenen Eingriffs wenigstens folgende Eingangsdaten (14) für die Unterstützungsfunktion enthalten
o wenigstens einen Bilddatensatz (10) der Bildüberwachung mit der Bildaufnahmeeinrichtung (33) und
o wenigstens einen strukturierten Textdatensatz (11), der wenigstens eine durchgeführte technische Bedienhandlung und/oder wenigstens eine eingriffsbezogene Eingriffsinformation beschreibt,
und wobei das Training derart erfolgt, dass bei Trainingsdaten bis zu einem Zeitpunkt enthaltenden Eingangsdaten (14) die Ausgangsdaten zumindest eine empfohlene Tätigkeit für die Bedienperson beschreiben und die empfohlene Tätigkeit die nächstfolgende Tätigkeit der Bedienperson gemäß den Trainingsdaten und/oder eine am besten bewertete Tätigkeit bei Einsatz von bestärkendem Lernen umfassen,
- Bereitstellen der trainierten Unterstützungsfunktion (15).

FIG 4

**Beschreibung**

Die Erfindung betrifft ein computerimplementiertes Verfahren, eine Bereitstellungseinrichtung, ein Computerprogramm und einen elektronisch lesbaren Datenträger zum Bereitstellen einer trainierten Unterstützungsfunktion für einen bildüberwachten medizinischen Eingriff an einer Eingriffsanordnung, die wenigstens eine technische Eingriffseinrichtung aufweist, von der wenigstens eine eine medizinische Bildaufnahmeeinrichtung zur Bildüberwachung des Eingriffs ist, zur Unterstützung einer Bedienperson. Daneben betrifft die Erfindung ein computerimplementiertes Verfahren, eine Steuereinrichtung, ein Computerprogramm und einen elektronisch lesbaren Datenträger zur Unterstützung einer Bedienperson bei einem bildüberwachten medizinischen Eingriff an einer Eingriffsanordnung, die die Steuereinrichtung und wenigstens eine technische Eingriffseinrichtung aufweist, von der wenigstens eine eine medizinische Bildaufnahmeeinrichtung zur Bildüberwachung des Eingriffs ist, und eine Eingriffsanordnung.

Bei medizinischen Eingriffen, beispielsweise minimalinvasive Untersuchungen und/oder Behandlungen, werden häufig technische Eingriffseinrichtungen zur Unterstützung einer den Eingriff durchführenden Person bzw. allgemein Bedienperson eingesetzt, die eine hohe Komplexität aufweisen können. Insbesondere werden derartige Eingriffe häufig mittels eines nicht-invasiven Bildgebungsverfahren bildüberwacht, sodass eine technische Eingriffseinrichtung eine entsprechende Bildaufnahmeeinrichtung sein kann. Beispielsweise können Röntgeneinrichtungen, insbesondere sogenannte Angiographieeinrichtungen bzw. C-Bogen-Röntgeneinrichtungen, eingesetzt werden, um den Eingriff fluoroskopisch zu überwachen, also mittels einer Zeitserie von Fluoroskopiebildern, die mit geringer Röntgendosis aufgenommen werden. Dabei kann beispielsweise eine Überlagerung mit vor dem medizinischen Eingriff aufgenommenen Vorabbilddaten erfolgen. Es wurde jedoch auch bereits vorgeschlagen, andere Bildaufnahmeeinrichtungen, beispielsweise Magnetresonanzeinrichtungen, Ultraschalleinrichtungen, Computertomographieeinrichtungen und dergleichen zur Bildüberwachung bei medizinischen Eingriffen einzusetzen.

Andere denkbare technische Eingriffseinrichtungen umfassen Mittel zur Unterstützung der Handhabung von beispielsweise minimalinvasiv in den Patienten eingebrachten medizinischen Instrumenten, wie beispielsweise Kathetern. Hierbei sind insbesondere sogenannte endovaskuläre Roboter bekannt, die die Bewegung von medizinischen Instrumenten im Blutgefäßsystem eines Patienten steuern bzw. unterstützen. Auch medizinische Eingriffsvorrichtungen, die die Verabreichung von Wirkstoffen steuern und/oder den Patienten anderweitig überwachen, sind bekannt.

Die Bedienung solcher technischer Eingriffseinrichtungen erfordert bereits sehr viel Grundwissen und Übung, insbesondere Expertise. Dies gilt insbesondere im Hinblick auf ihre effiziente und erfolgreiche Nutzung im Rahmen eines bestimmten medizinischen Eingriffs, also in Wechselwirkung mit der speziellen, patientenspezifischen Eingriffssituation. Doch auch allgemein ist die Komplexität und somit das benötigte Wissen für die möglichst erfolgreiche Durchführung medizinischer Eingriffe äußerst hoch. Insbesondere für das allgemeine Einschätzen von Eingriffssituationen benötigt die Expertise des ärztlichen Personals üblicherweise große praktische Erfahrung eigener manueller Durchführung, was häufig als "Learning by Doing" bezeichnet wird.

Ein Fachgebiet, in dem diese Erfahrung durch das praktische, manuelle Durchführen ("handson experience") von medizinischen Eingriffen besondere Wichtigkeit besitzt, ist die Neurologie, bei der Bildüberwachung konkret die Neuroradiologie. Komplexe Prozeduren, beispielsweise das sogenannte "Coiling" von Aneurysmen, erfordern ein hohes Niveau an Fähigkeiten und Präzision, das nur durch wiederholte Praxis erlangt werden kann. Beispielsweise muss man, um ein besserer Neuroradiologe zu werden, häufig Prozeduren wie Aneurysma-Coiling durchführen. Der durchschnittliche Neuroradiologe hat jedoch nicht die Möglichkeit, eine große Menge solcher Prozeduren jährlich durchzuführen, was die Entwicklung seiner Fähigkeiten behindern kann.

Im Ergebnis werden ältere oder anderweitig erfahrene Personen, die medizinische Eingriffe durchführen (beispielsweise Neuro-Interventionalisten), aufgrund ihrer großen Praxiserfahrung als besser angesehen. Sie hatten mehr Gelegenheit, diese schwierigen und herausfordernden Prozeduren durchzuführen, sodass sie ihre Fähigkeiten verfeinert haben und eine Vielzahl klinischer Szenarien besser handhaben können. Dies ist äußerst wichtig, da ein reales Risiko für Patienten besteht, an denen derartige medizinische Eingriffe durchgeführt werden, sodass das Fähigkeitsniveau der den Eingriff durchführenden Person das Ergebnis des Eingriffs auf signifikante Weise beeinflusst. Daher kann die Erfahrung, die mit dem Alter kommt, in überlegenen Fähigkeiten bei medizinischen Eingriffen, beispielsweise in der Neuroradiologie, resultieren.

Die Unterstützung jüngerer Interventionalisten ist aus mehreren Gründen wichtig. Zum einen altert die aktuelle Generation von Interventionalisten, sodass eine neue Generation rekrutiert und trainiert werden muss, um fortgesetzte medizinische Versorgung sicherzustellen. Darüber hinaus ist die Lernkurve bei medizinischen Eingriffen, insbesondere minimalinvasiven Eingriffen, steil und das Risiko für Patienten ist signifikant. Mithin müssen jüngere, Eingriffe durchführende Personen mit hinreichenden Gelegenheiten zur praktischen Durchführung der Eingriffe, Betreuung durch erfahrene Kollegen und Zugang zu fortbestehenden Lernmöglichkeiten versorgt werden. Schließlich ist es auch wichtig, hinreichend viele Arbeitskräfte im Feld der medizinischen Eingriffe zu halten, indem die Bedürfnisse des Nachwuchses berücksichtigt werden und ihnen Unterstützung zuteil wird.

Auch die technischen Gegebenheiten, mithin die technischen Eingriffseinrichtungen einer entsprechenden Eingriffsanordnung, spiegeln die Komplexität im Umfeld medizinischer Eingriffe wider. Beispielsweise sind die Bildaufnahmeeinrichtungen, beispielsweise Angiographieeinrichtungen, komplexe medizinische Einrichtungen, die entworfen sind, eine große Auswahl an Features und Optionen bereitzustellen, um die unterschiedlichen Wünsche der Bedienpersonen und/oder die unterschiedlichen Anforderungen für verschiedene Eingriffe abzudecken. Diese Komplexität führt zu einer herausfordernden Bedienung und Interaktion mit den Eingriffseinrichtungen, insbesondere für jene, die neu mit der Eingriffsanordnung arbeiten oder denen die Funktionalitäten nicht vollständig bekannt sind.

In einer ersten Problematik kann die schiere Anzahl der Features und Optionen, die verfügbar sind, überwältigend sein. Die Eingriffseinrichtungen sind ausgelegt, um eine Mehrzahl von medizinischen Eingriffen zu unterstützen, von denen jeder seine eigenen spezifischen Anforderungen hat. Das bedeutet, eine Vielzahl von Einstellungsmöglichkeiten und Parametern, die angepasst werden können, können zur Verwirrung und Verunsicherung der Bedienpersonen führen.

Ein zweites Feld, in dem Probleme entstehen können, sind die komplexen Benutzerschnittstellen der Eingriffseinrichtungen. Aufgrund der höheren Risiken bei medizinischen Eingriffen werden Eingriffseinrichtungen so ausgelegt, dass ein hohes Niveau an Kontrolle für die Bedienperson bereitgestellt wird. Dies kann jedoch in schwer verständlichen, dicht gefüllten Benutzerschnittstellen resultieren, die ohne extensives Training und große Erfahrung schwer zu navigieren sein können.

Schließlich sind die Eingriffseinrichtungen oft integriert mit anderen Geräten und Software ausgebildet, sodass eine zusätzliche Komplexitätsschicht entsteht. Beispielsweise können die Eingriffseinrichtungen mit Patientenüberwachungssystemen, Bildverarbeitungssoftware und anderen medizinischen Einrichtungen verbunden sein. Das Verständnis für die Interaktion zwischen diesen Komponenten kann herausfordernd sein.

Erfahrene Bedienpersonen, also Experten-Benutzer, sind äußerst erfahren in der Nutzung aller Features und Optionen der Eingriffseinrichtungen. Die meisten anderen Bedienpersonen nutzen jedoch nur einen Bruchteil der verfügbaren Möglichkeiten. Dies liegt hauptsächlich darin begründet, dass sie von der Existenz einer speziellen Option nichts wissen oder sich an diese während des medizinischen Eingriffs nicht oder nicht im Detail erinnern können, wobei aufgrund des Zeitdrucks beim medizinischen Eingriff keine Möglichkeit besteht, Bedienungsanleitungen und dergleichen zu konsultieren.

Bislang sind diesbezüglich für die Bedienpersonen hauptsächlich Bedienungsanleitungen und Lehrveranstaltungen neben eigener persönlicher Erfahrung verfügbar, um die geeignetsten Bedienhandlungen an den Eingriffseinrichtungen während eines medizinischen Eingriffs auffinden zu können.

Der Erfindung liegt daher die Aufgabe zugrunde, eine Möglichkeit zur hochqualitativen Unterstützung von Bedienpersonen bei medizinischen Eingriffen anzugeben.

Diese Aufgabe wird erfindungsgemäß gelöst durch die Verfahren, Einrichtungen, Computerprogramme, elektronisch lesbaren Datenträger und die Eingriffsanordnung gemäß den nebengeordneten Patentansprüchen. Vorteilhafte Weiterbildungen ergeben sich aus den Unteransprüchen.

Im Folgenden wird die Lösung gemäß der vorliegenden Erfindung mit Bezug auf die beanspruchten Verfahren, Einrichtungen, Computerprogramme, elektronisch lesbaren Datenträger und die Eingriffsanordnung beschrieben. Merkmale, Vorteile oder alternative Ausgestaltungen, die hierin beschrieben sind, können auf die anderen beanspruchten Gegenstände sinngemäß übertragen werden und umgekehrt. Mit anderen Worten können Ansprüche und Ausführungsbeispiele für die Einrichtungen mit Merkmalen, die im Kontext der entsprechenden Verfahren beschrieben oder beansprucht sind, weitergebildet werden und umgekehrt, wobei Entsprechendes auch für die Computerprogramme, die elektronisch lesbaren Datenträger und die Eingriffsanordnung gilt. Im Fall der Übertragung vom Verfahren auf eine Einrichtung werden die funktionalen Merkmale des Verfahrens als physikalische Funktionseinheiten der Einrichtung implementiert.

Insbesondere wird die Erfindung im Folgenden sowohl (und zunächst) in Bezug auf ein Verfahren zur Bereitstellung einer Unterstützungsfunktion (Bereitstellungsverfahren bzw. Trainingsverfahren), eine Bereitstellungseinrichtung, ein Bereitstellungscomputerprogramm sowie einen entsprechenden elektronisch lesbaren Datenträger als auch in Bezug auf ein Verfahren zur Unterstützung einer Bedienperson (Unterstützungsverfahren bzw. Anwendungsverfahren), eine Steuereinrichtung (zur Unterstützung der Bedienperson), die Eingriffsanordnung, ein Unterstützungscomputerprogramm sowie einen entsprechenden elektronisch lesbaren Datenträger beschrieben. Auch diesbezüglich können Merkmale, Eigenschaften und Ausführungsbeispiele sinngemäß zwischen Bereitstellung, insbesondere Training, und Unterstützung übertragen werden. Insbesondere können Daten, die in den jeweiligen Verfahren, Einrichtungen, Computerprogrammen und zugeordneten Datenträgern zur Bereitstellung verwendet werden, dieselben Eigenschaften und Merkmale wie entsprechende Daten in den Verfahren, Einrichtungen, Computerprogrammen und zugeordneten Datenträgern zur Unterstützung haben und umgekehrt.

In einem erfindungsgemäßen, computerimplementierten Verfahren zum Bereitstellen einer trainierten Unterstützungsfunktion für einen bildüberwachten medizinischen Eingriff an einer Eingriffsanordnung, die wenigstens eine technische Eingriffseinrichtung aufweist, von der wenigstens eine eine medizinische Bildaufnahmeeinrichtung zur Bildüberwachung des Eingriffs ist, zur Unterstützung einer Bedienperson, sind folgende Schritte vorgesehen:

- Bereitstellen einer für mehrere Datentypen, umfassend zwei- und/oder dreidimensionale Bilder und Videos und strukturierten Text, vortrainierten generativen Funktion, die aus Eingangsdaten der Datentypen auf die Eingangsdaten bezogene Ausgangsdaten generiert, als untrainierte Unterstützungsfunktion,

- Trainieren der Unterstützungsfunktion mit auf den bildüberwachten medizinischen Eingriff bezogenen Trainingsdatensätzen, wobei die Trainingsdatensätze für mehrere Zeitpunkte einer Eingriffsdauer jedes in den Trainingsdatensätzen beschriebenen Eingriffs wenigstens folgende Eingangsdaten für die Unterstützungsfunktion, die den Ablauf des Eingriffs bis zu dem jeweiligen Zeitpunkt beschreiben, enthalten wenigstens einen Bilddatensatz der Bildüberwachung mit der Bildaufnahmeeinrichtung und wenigstens einen strukturierten Textdatensatz, der wenigstens eine durchgeführte technische Bedienhandlung und/oder wenigstens eine eingriffsbezogene, insbesondere bildgebungsbezogene, Eingriffsinformation beschreibt und seitens wenigstens einer der wenigstens einen Eingriffseinrichtung vorliegt, und wobei das Training derart erfolgt, dass bei Trainingsdaten bis zu einem Zeitpunkt enthaltenden Eingangsdaten die Ausgangsdaten zumindest eine empfohlene Tätigkeit für die Bedienperson beschreiben und die empfohlene Tätigkeit die nächstfolgende Tätigkeit der Bedienperson gemäß den Trainingsdaten und/oder eine am besten bewertete Tätigkeit bei Einsatz von bestärkendem Lernen umfassen,

- Bereitstellen der trainierten Unterstützungsfunktion.

Der Erfindung liegt also der Gedanke zugrunde, ein domänenspezifisch trainiertes, generatives Maschinenlern-Modell, mithin die trainierte Unterstützungsfunktion, bereitzustellen und sodann zu nutzen, um einer einen medizinischen Eingriff durchführenden Person, konkret der Bedienperson, folgende Schritte und Maßnahmen, insbesondere umfassend Bedienhandlungen für die technischen Eingriffseinrichtungen, zu empfehlen und gegebenenfalls auch hinreichend zu erläutern. Dabei wird mithin ausgenutzt, dass vortrainierte generative, auf großen Datenmengen basierende Funktionen, die insbesondere für bestimmte Datentypen als Eingangsdaten vortrainiert sind, bereits verfügbar sind und domänenspezifisch so angepasst werden können, dass mit einem insbesondere multimodalen Eingangsdatensatz, der bevorzugt durch die technischen Eingriffseinrichtungen, insbesondere vollständig, bereitgestellt wird und somit keinerlei zusätzliche, aktuelle benutzerseitige Information benötigt, die aktuelle Eingriffssituation beurteilt und eine Tätigkeitsempfehlung als Ausgangsdaten ausgegeben werden kann. Dabei kann in zweckmäßigen Ausführungsbeispielen vorgesehen sein, dass die generative Funktion ein Large Language Model (LLM) ist oder umfasst. Dies ermöglicht es insbesondere, die Ausgangsdaten sogleich in unmittelbar an die Bedienperson weitergebbarer, verständlicher Form zu generieren. Ein Beispiel für eine vortrainierte generative Funktion, die im Rahmen der vorliegenden Erfindung verwendet werden kann, ist unter dem Handelsnamen "Google Gemini" bekannt.

Dabei sei bereits an dieser Stelle angemerkt, dass die Ausgangsdaten jeweils mehrere Tätigkeiten mit einer zugeordneten Empfehlungsinformation, die die Stärke der Empfehlung beschreibt, enthaltend ermittelt werden können. Beispielsweise kann also eine Liste von jeweils eine Tätigkeit beschreibenden Ausgangsdatenblöcken mit zugeordneten Empfehlungsinformationen, beispielsweise Bewertungsinformationen, erzeugt werden. Denkbar ist es jedoch auch, dass die Ausgangsdaten als generierter Text- und/oder Bildinhalt insgesamt ausgegeben werden und die mehreren Tätigkeiten mit den zugeordneten Empfehlungsinformationen enthalten.

Im Allgemeinen bildet eine trainierte Funktion kognitive Funktionen ab, die Menschen mit anderen menschlichen Gehirnen assoziieren. Durch Training basierend auf Trainingsdaten (Maschinenlernen) ist die trainierte Funktion in der Lage, sich an neue Umstände anzupassen und Muster zu detektieren und zu extrapolieren. Ein anderer Ausdruck für "trainierte Funktion" ist "trainiertes Maschinenlernmodell".

Allgemein gesagt können Parameter einer trainierten Funktion durch Training angepasst werden. Insbesondere können überwachtes Lernen, halbüberwachtes Lernen, nicht überwachtes Lernen, bestärkendes Lernen (Reinforcement Learning) und/oder aktives Lernen verwendet werden. Darüber hinaus kann auch Repräsentationslernen (auch als "feature learning" bekannt) eingesetzt werden. Die Parameter der trainierten Funktion können insbesondere iterativ durch mehrere Trainingsschritte angepasst werden. Insbesondere kann bei dem Training eine bestimmte Kostenfunktion minimiert werden. Beispielsweise kann beim Training eines neuronalen Netzes der Backpropagation-Algorithmus eingesetzt werden.

Eine trainierte Funktion kann beispielsweise ein neuronales Netz, eine Support Vector Machine (SVM), einen Entscheidungsbaum und/oder ein Bayes-Netzwerk umfassen und/oder die trainierte Funktion kann auf k-means-Clustering, Q-Learning, genetischen Algorithmen und/oder Zuordnungsregeln basieren. Insbesondere kann ein neuronales Netzwerk ein tiefes neuronales Netzwerk, ein Convolutional Neural Network (CNN) oder ein tiefes CNN sein. Darüber hinaus kann das neuronale Netzwerk ein Adversarial Network, ein tiefes Adversarial Network und/oder ein Generative Adversarial Network (GAN) sein.

Mit besonderem Vorteil umfasst die trainierte Unterstützungsfunktion ein Convolutional Neural Network (CNN). Ein Convolutional Neural Network (CNN) ist ein neuronales Netz, dass eine Faltungsoperation anstatt allgemeiner Matrixmultiplikation in wenigstens einer seiner Schichten, der sogenannten Faltungsschicht, verwendet. Insbesondere kann eine Faltungsschicht ein Skalarprodukt eines oder mehrerer Faltungskerne mit den eingehenden Daten/Bildern der Faltungsschicht durchführen, wobei die Einträge des einen oder der mehreren Faltungskerne die Parameter oder Gewichte sind, die durch Training angepasst werden. Insbesondere können das innere Frobenius-Produkt und die ReLu-Aktivierungsfunktion verwendet werden. Ein CNN kann zusätzliche Schichten, beispielsweise Pooling-Schichten, vollständig verbundene Schichten und Normalisierungsschichten umfassen.

Durch CNN können insbesondere Billddatensätze der Eingangsdaten auf äußerst effiziente Weise verarbeitet werden, da eine Faltungsoperation basierend auf unterschiedlichen Kernen verschiedenste Bildmerkmale extrahieren kann, sodass durch Anpassung der Gewichte der Faltungskern die relevanten Bildmerkmale während des Trainings aufgefunden werden können. Darüber hinaus müssen basierend auf dem Sharing der Gewichte in den Faltungskernen weniger Parameter trainiert werden, sodass ein Overfitting in der Trainingsphase vermieden wird und schnelleres Training oder eine größere Anzahl an Schichten in dem CNN erlaubt werden, sodass die Leistungsfähigkeit des Netzwerks erhöht wird.

Ein Kern der Erfindung ist es, mittels der Unterstützungsfunktion eine Möglichkeit bereitzustellen, weniger erfahrene Bedienpersonen, beispielsweise Neuroradiologen, bei medizinischen Eingriffen zu unterstützen und hierbei Fachwissen erfahrener Experten weiterzugeben. Die Trainingsdaten umfassen hierzu bevorzugt Informationen zum Ablauf kompletter medizinischer Eingriffe, beispielsweise einer bestimmten, zu unterstützenden Gattung von medizinischen Eingriffen, die von Experten durchgeführt oder zumindest beurteilt sind. Zum Erhalt der Trainingsdaten kann ausgenutzt werden, dass viele technische Eingriffseinrichtungen bereits ausgestaltet sind, aufgenommene Bilddatensätze und/oder strukturierte Textdatensätze, beispielsweise Log-Dateien, Konfigurationsdateien und dergleichen, für Eingriffe zu speichern und beispielsweise für eine Datenauswertung zur Verbesserung der technischen Eingriffseinrichtungen bereitzustellen. Dieser große Datenpool kann mithin genutzt werden, um geeignete Trainingsdaten, auch in größerer Menge, daraus zu extrahieren, wobei insbesondere eine Auswahl aus dem Datenpool, beispielsweise einer herstellerseitig vorgehaltenen Datenbank, nach Erfahrungsgrad der jeweiligen wenigstens einen Bedienperson und/oder Eingriffserfolg ("clinical outcome") erfolgen kann. Hierbei kann in besonders vorteilhaften Ausgestaltungen der vorliegenden Erfindung ausgenutzt werden, dass zumindest teilweise Tätigkeiten der Bedienperson bei dem medizinischen Eingriff erfasst sind, was insbesondere für technische Bedienhandlungen an den Eingriffseinrichtungen, beispielsweise der Bildaufnahmeeinrichtung, gilt. Somit ist aber, wenn die als vortrainierte generative Funktion bereitgestellte Unterstützungsfunktion mit Eingangsdatensätzen bis zu einem bestimmten Zeitpunkt trainiert wird, vorteilhaft zumindest teilweise eine Grundwahrheit bekannt, nämlich die zum nächsten Zeitpunkt bzw., insbesondere bei medizinischen Eingriffsoptionen und deren Empfehlung, allgemein im weiteren Verlauf des Eingriffs durchgeführte Tätigkeit der Bedienperson. Ist diese nicht bekannt, wird vorgeschlagen, bestärkendes Lernen einzusetzen, beispielsweise indem die Ausgangsdaten von einem Experten bewertet werden. Das bestärkende Lernen wird bevorzugt lediglich zusätzlich eingesetzt.

Besonders eignet sich die Bereitstellung der Unterstützungsfunktion für Klassen von medizinischen Eingriffen, in denen eine besonders hohe Komplexität vorhanden ist, die sich sowohl auf die medizinischen Sachverhalte als auch auf die Eingriffsanordnung und deren Bedienung beziehen kann. Ein Beispiel hierfür sind neuroradiologische Eingriffe, beispielsweise die Behandlung von Aneurysmen, wobei ein Training der Unterstützungsfunktion auch ganz konkrete Eingriffsarten betreffen kann, um möglichst robuste Ergebnisse zu erzielen. Derartige konkrete Eingriffsarten im Bereich der Neuroradiologie können beispielsweise "Aneurysma-Coiling", Embolisationen von krankhaft veränderten Gefäßen und dergleichen sein.

Durch die Unterstützungsfunktion kann vorteilhafter Weise nicht nur weniger erfahrenen Bedienpersonen Expertenwissen fallbezogen zugänglich gemacht werden, um diese zu unterstützen und/oder auszubilden, sondern die Bedienperson wird zur optimalen Behandlung des Patienten und/oder zur optimalen Nutzung der technischen Eingriffseinrichtungen angeleitet. Die Geschwindigkeit der Durchführung von medizinischen Eingriffen kann erhöht werden und es besteht das Potenzial zur Verbesserung des klinischen Ergebnisses der medizinischen Eingriffe.

Vorteilhafte Weiterbildungen der Erfindung sehen vor, dass der strukturierte Textdatensatz der Trainingsdaten eine Log-Datei der wenigstens einen der wenigstens einen Eingriffseinrichtung, die Bedienhandlungen und/oder eingestellte Betriebsparameter beschreibt, und/oder eine Konfigurationsdatei, die Bildaufnahmeparameter und/oder Eingriffsparameter für die Bildaufnahmeeinrichtung enthält, und/oder ein Anteil einer solchen Konfigurationsdatei, insbesondere ein Metadatensatz, ist oder umfasst. Log-Dateien sind grundsätzlich bereits bekannt und protokollieren insbesondere wenigstens Bedienhandlungen, bevorzugt auch automatische Steuermaßnahmen. Zudem können in Log-Dateien auch eingestellte Betriebsparameter der jeweiligen Eingriffseinrichtung gespeichert sein. Die Log-Dateien werden üblicherweise strukturiert gespeichert, gelten mithin als strukturierte Textdatensätze, deren Struktur durch die Unterstützungsfunktion entsprechend genutzt werden kann. Metadatensätze sind in der medizinischen Bildgebung beispielsweise im DICOM-Standard bekannt, so dass der Metadatensatz ein DICOM-Metadatensatz sein kann. Neben Betriebsparametern der Bildaufnahmeeinrichtung, insbesondere Bildaufnahmeparametern, können darin auch Informationen zum medizinischen Eingriff bzw. dem Patienten selbst, also der konkreten Eingriffssituation, enthalten sein. Durch die Nutzung strukturierter Textdatensätze wird im Allgemeinen auf Bezugnahme auf nicht strukturierten Text, der durch künstliche Intelligenz schwerer zu interpretieren ist, verzichtet. Dies ist der Robustheit der resultierenden trainierten Unterstützungsfunktion zuträglich.

Ferner kann hinsichtlich der Trainingsdaten vorgesehen sein, dass der wenigstens eine Bilddatensatz eine Zeitserie von Fluoroskopiebildern und/oder einen dreidimensionalen, aus zweidimensionalen Projektionsbildern rekonstruierten Volumendatensatz umfasst und/oder die Trainingsdaten und die Eingangsdaten für wenigstens einen Teil der enthaltenen Eingriffe ferner einen Vorabbilddatensatz, der vor dem Eingriff mit der oder einer weiteren Bildaufnahmeeinrichtung aufgenommen wurde, umfassen. Insbesondere enthält eine Zeitserie von Fluoroskopiebildern oder sonstigen, den Verlauf des Eingriffs dokumentierenden Bilddatensätzen auch, insbesondere zusätzliche, Information über den Ablauf des Eingriffs und/oder durchgeführte Tätigkeiten, insbesondere Maßnahmen. Vorabbilddatensätze liefern Zusatzinformationen zum Eingriff im Allgemeinen. Zweckmäßig ist der Einsatz einer Angiographieeinrichtung bzw. einer Röntgeneinrichtung mit einem C-Bogen, an dem sich gegenüberliegend ein Röntgenstrahler und ein Röntgendetektor befestigt sind. Besonders bevorzugt sind die Bilddatensätze und strukturierten Textdateien auch jene, die bei der Inferenz, also Anwendung der trainierten Unterstützungsfunktion zur Unterstützung einer Bedienperson, als Eingangsdaten verwendet werden.

Neben der Bildaufnahmeeinrichtung kann als wenigstens eine weitere technische Eingriffseinrichtung auch ein wenigstens Roboter, der die Bewegung eines medizinischen Instruments steuert und/oder dessen Handhabung unterstützt, vorgesehen sein. Hierbei kann es sich beispielsweise um einen endovaskulären Roboter (Endovascular Robot, EVR) handeln. Auch technische Eingriffseinrichtungen sind zusätzlich oder alternativ selbstverständlich denkbar.

Mit besonderem Vorteil, wie bereits dargelegt, kann vorgesehen sein, dass als Eingangsdaten für die Unterstützungsfunktion ausschließlich technisch von der wenigstens einen Eingriffseinrichtung verfügbare Datensätze und/oder Dateien verwendet werden. Hierdurch kann auf zusätzliche Benutzereingaben während des medizinischen Eingriffs verzichtet werden. Es werden also technisch verfügbare, objektiv physikalisch-technische Sachverhalte beschreibende Eingangsdaten, die technisch erfasst werden, ausgewertet, um eine Empfehlung für den weiteren Fortgang des medizinischen Eingriffs abzuleiten. Die Bilddatensätze sowie die strukturierten Textdatensätze werden von den technischen Eingriffseinrichtungen über entsprechende Schnittstellen bereitgestellt, beispielsweise einer übergeordneten, den Betrieb der Eingriffseinrichtungen sowie gegebenenfalls weiterer Komponenten zentral koordinierenden Steuereinrichtung, die auch die trainierte Unterstützungsfunktion bei einem medizinischen Eingriff anwenden kann. Hierbei werden für jeden Eingriff insbesondere die Bilddatensätze und/oder strukturierten Textdateien, falls sie nicht ohnehin den Eingriff bereits bis zum aktuellen Zeitpunkt abdecken, in der praktischen Anwendung gesammelt und zu entsprechenden Eingangsdatensätzen zusammengefasst.

Die Eingangsdaten sind bevorzugt multimodal. Das bedeutet, es werden Informationen verschiedener Quellen zusammengeführt und durch die trainierte Unterstützungsfunktion ausgewertet. Multimodal ist hierbei insbesondere so zu verstehen, dass die Eingangsdaten von wenigstens zwei unterschiedlichen technischen Eingriffseinrichtungen stammen und/oder die Bilddaten mit wenigstens zwei Bildaufnahmemodalitäten und/oder Bildgebungsverfahren ermittelt worden sind.

In besonders bevorzugter Weiterbildung der vorliegenden Erfindung kann vorgesehen sein, dass bei dem Training und/oder im Rahmen einer zusätzlich bereitgestellten Überprüfungsfunktion zur Überprüfung der Ausgangsdaten wenigstens eine durch Ausgangsdaten beschriebene Tätigkeit mittels wenigstens einer Ausschlussbedingung ausgeschlossen und/oder aus den Ausgangsdaten entfernt wird. Derartige Ausschlussbedingungen, die sowohl beim Training als auch bei der Interferenz im tatsächlichen Einsatz der bereitgestellten Unterstützungsfunktion Relevanz entfalten, können auch als "Guard rails", also Schutzplanke bzw. Leitplanke, verstanden werden und explizit oder implizit umgesetzt werden. Auf diese Weise kann vermieden werden, dass ungewollte Interaktionen bzw. allgemein Tätigkeiten, die beispielsweise Risiken für den Patienten mit sich bringen, allgemein unsinnig sind oder für die technischen Eingriffseinrichtungen und/oder deren Nutzung nachteilhaft, als Empfehlung verhindert werden können.

Konkret kann in diesem Zusammenhang vorgesehen sein, dass der Ausschluss für wenigstens eine der wenigstens einen Ausschlussbedingung in Abhängigkeit eines Eingangsdatensatzes und/oder eines zugehörigen Ausgangsdatensatzes einer Anwendung der Unterstützungsfunktion und/oder für wenigstens eine der wenigstens einen Ausschlussbedingung absolut erfolgt.

Der erstgenannte Fall kann sich beispielsweise im Hinblick auf Eingangsdaten

- auf medizinisch für den speziellen Fall zu vermeidende Maßnahmen und/oder
- das Erreichen eines technischen Grenzwerts, beispielsweise für eine Strahlendosis, aufgrund der Historie des Eingriffs, etc.;
und/oder im Hinblick auf die Ausgangsdaten

- auf eine Begrenzung der Stärke von Maßnahmen und/oder Änderungen bei diesen betreffenden Tätigkeiten

beziehen. Die Ausschlussbedingung kann tätigkeitsspezifisch sein oder auch von Eingangsdaten des Eingangsdatensatzes abhängen, z.B. keine zu hohen Röntgendosen, keine zu langen Bewegungswege, keine Bewegung aus dem Eingriffsbereich und dergleichen.

Der zweitgenannte Fall betrifft allgemein nicht zu empfehlende Maßnahmen bzw. Tätigkeiten, beispielsweise das Herunterfahren einer technischen Eingriffseinrichtung und/oder einer die trainierte Unterstützungsfunktion durchführenden Steuereinrichtung, grundsätzlich (beispielsweise aufgrund einer Kollision mit einer ortsfesten Komponente der Eingriffsanordnung) nicht einzunehmende Stellungen einer Einrichtung, und dergleichen. Allgemein gesprochen können Ausschlussbedingungen auch solche Tätigkeiten betreffen, die eine Experten-Bedienperson nicht durchführen würde.

Die Ausschlussbedingungen können in das Training selbst eingebracht werden. Beim Training können Ausschlussbedingungen auch aufgrund einer Benutzereingabe definiert werden, insbesondere als Erweiterung oder Spezialfall des bereits beschriebenen bestärkenden Lernens, wo beispielsweise ein Experte oder eine sonstige menschliche Überprüfungsperson Tätigkeiten als (für diesen Fall oder allgemein) nicht zu empfehlen markieren können. Die Unterstützungsfunktion lernt dann, solche Tätigkeiten als Ausgangsdaten zu vermeiden.

Die Ausschlussbedingungen können zusätzlich oder alternativ auch in einer zusätzlichen Überprüfung der Ausgangsdaten realisiert werden. Die Überprüfungsfunktion kann in Ausführungsbeispielen selbst ein maschinenlehrbares Modell sein und/oder im Rahmen des oder eines weiteren Trainings trainiert werden. In diesem Fall werden die Ausschlussbedingungen also durch die Parameter des Modells definiert und durch diese implizit umgesetzt. Auch eine einfachere Umsetzung der Überprüfungsfunktion ist selbstverständlich denkbar, beispielsweise als Liste auszuschließender Tätigkeiten.

Eine Entfernung von Tätigkeiten aus den Ausgangsdaten aufgrund einer Ausschlussbedingung kann insbesondere dann erfolgen, wenn diese mehrere Tätigkeiten vorschlägt.

Eine erste Gruppe von Ausführungsbeispielen der Erfindung kann vorsehen, dass bei der Ermittlung von Ausgangsdaten, die eine auf den Eingriff selbst bezogene medizinische Tätigkeit beschreiben, in dem Trainingsvorgang zusätzlich zu wenigstens einem Teil der Eingangsdatensätze eine Experteninformation, die medizinisches Expertenwissen zu der durch den Eingangsdatensatz beschriebenen Eingriffssituation beschreibt, verwendet wird. Mit anderen Worten kann vorgesehen sein, dass die Unterstützungsfunktion dazu trainiert und verwendet wird, Untersuchungs- und/oder Behandlungsoptionen und/oder sonstige, unmittelbar auf den Eingriff selbst bezogene medizinische Tätigkeiten vorzuschlagen. Dabei kann die Ausgestaltung gezielt ausschließlich auf medizinische Tätigkeiten gerichtet werden, denkbar ist es aber auch, die Unterstützung auf andere Tätigkeiten, beispielsweise Bedienhandlungen der noch zu diskutierenden zweiten Gruppe von Ausführungsbeispielen, zu erweitern. Die von der Unterstützungsfunktion vorgeschlagene Tätigkeit kann sich beispielsweise auf die Nutzung von Instrumenten, Implantaten und/oder anderen medizinischen Hilfsmitteln und/oder auf die Gabe von medizinisch relevanten Wirksubstanzen beziehen, aber auch auf die beispielsweise in den Bilddatensätzen sichtbare Anatomie des Patienten. Hierbei handelt es sich bei den Ausgangsdaten grundsätzlich lediglich um Empfehlungen, die selbstverständlich von der Bedienperson noch zu beurteilen sind. Werden sie in Textform bestimmt, sind sie zweckmäßig auch entsprechend formuliert, beispielsweise der Art "Ziehen Sie in Betracht, eine weitere Spirale zu verwenden" oder "Experten haben in einer ähnlichen Situation eine Verwendung des Alternativmittels in Betracht gezogen". Insbesondere im Hinblick auf die Ausbildung, Weiterbildung und Ergänzung der Erfahrung neuer bzw. junger Interventionalisten ist damit eine optimale Unterstützung gegeben, die sich auf den Eingriff im Ganzen bezieht.

Beim domainspezifischen Training der Unterstützungsfunktion muss diese mithin lernen, anhand der Eingangsdaten die aktuelle Eingriffssituation hinsichtlich anstehender und sinnvoller medizinischer Tätigkeiten zu beurteilen, was insgesamt als Situationsverständnis oder "scenic understanding" bezeichnet werden kann. Die trainierte Unterstützungsfunktion soll dann in der Lage sein, an einem bestimmten Zeitpunkt des medizinischen Eingriffs zu prädizieren, was eine Experten-Bedienperson als nächstes tun würde. Um das Situationsverständnis, mithin das Verständnis dafür, was klinisch geschieht, zu erhalten, können die Trainingsdaten Expertenwissen enthalten bzw. dieses kann beim Training der Unterstützungsfunktion hinzugefügt werden.

Es kann mithin vorgesehen sein, dass die Experteninformation wenigstens teilweise eine von einem Experten vorgeschlagene und/oder bei der Aufzeichnung des Eingangsdatensatzes durchgeführte medizinische Solltätigkeit beschreibt, die als Grundwahrheit verwendet wird. Die Experteninformation kann insbesondere Teil der Trainingsdaten bilden. Konkret kann die Experteninformation wenigstens teilweise aus einer benutzerseitigen Annotation und/oder unter Verwendung einer mittels Expertenwissen vorab trainierten Expertisefunktion, die insbesondere wenigstens die Bilddaten eines jeweiligen Eingangsdatendatzes als Eingangsdaten nutzt, ermittelt werden.

Eine erste Option ist es mithin, die Bilddatensätze und strukturierten Textdatensätze der Trainingsdaten durch Annotation unmittelbar um die Informationen zu medizinischen Tätigkeiten zu ergänzen, die sich aus den Bilddatensätzen und den strukturierten Textdatensätze nicht bzw. nicht eindeutig herleiten lassen. Hierzu kann beispielsweise eine nicht automatisch durchgeführte, während und/oder nach dem Eingriff durchgeführte Protokollierung herangezogen werden, die vorzugsweise in den Trainingsdaten als Annotationen ergänzt wird. Möglich ist selbstverständlich auch, die Bilddatensätze und gegebenenfalls auch strukturierten Textdatensätze wenigstens einer Expertenperson vorzulegen, die die Annotationen als Experteninformation nachträglich hinzufügt.

Möglich ist es in einer zweiten Option aber auch, eine bereits vorab trainierte Expertisefunktion einzusetzen, die zur Beurteilung der klinischen Eingriffssituation ausgebildet ist. Beispielsweise kann eine derartige trainierte Expertisefunktion mit Bilddaten als Eingangsdaten arbeiten, die die Bilddatensätze, die auch als Eingangsdaten für die Unterstützungsfunktion dienen sollen, umfassen. Auf diese Weise wird also bereits geleistete Arbeit genutzt, um die Experteninformation, insbesondere als Teil der Trainingsdaten, zusammenzustellen.

Die erste und die zweite Option können vorteilhaft auch kombiniert werden, beispielsweise, wenn die Expertisefunktion zunächst eine Zwischeninformation zum Verständnis der Eingriffssituation liefert, welche dann von einer Expertenperson berücksichtigt wird, um die Experteninformation zusammenzustellen, welche insbesondere die nächstfolgende medizinische Tätigkeit betrifft.

Schließlich kann alternativ oder zusätzlich im Rahmen einer dritten Option auch vorgesehen sein, dass die Experteninformation im Rahmen des bestärkenden Lernens als Bewertung des einem jeweiligen Eingangsdatensatz zugehörigen Ausgangsdatensatzes bereitgestellt wird. Diese Bewertung kann bevorzugt auf einer Benutzereingabe einer Expertenperson basieren. Es wird mithin eine Benutzereingabe verwendet, um die Leistungsfähigkeit der trainierten Unterstützungsfunktion zu verbessern. Insbesondere im Vergleich zu einer Expertisefunktion, die in ihrer Verlässlichkeit eingeschränkt sein kann, wird diese Variante zusätzlich oder alternativ bevorzugt, da ungeeignete Ausgangsdaten von der Expertenperson unmittelbar identifiziert und entsprechend ausgeschlossen bzw. schlecht bewertet werden können.

Insbesondere im Kontext der Prädiktion medizinischer Tätigkeiten, gegebenenfalls aber auch allgemein, kann eine zweckmäßige Ausgestaltung vorsehen, dass die Ausgangsdaten einen Verweis auf wenigstens eine Referenzinformation, insbesondere der Trainingsdatensätze, die einen vergleichbaren Eingriff betrifft, umfassend ermittelt werden. Sind mithin die Trainingsdaten zumindest teilweise auch bei Anwendung der trainierten Unterstützungsfunktion noch, beispielsweise in einer mit einer die Unterstützungsfunktion anwendenden Steuereinrichtung verbundenen Datenbank, verfügbar, kann ein Teil der Ausgangsdaten einen Verweis auf einen vergleichbar ablaufenden Eingriff, der durch die Trainingsdaten beschrieben wird, und/oder eine vergleichbare Eingriffssituation umfassen, wobei der Verweis entsprechend genutzt werden kann, um geeignete Zusatzinformationen, auf die der Verweis abzielt, auszugeben, beispielsweise, worauf im Folgenden noch näher eingegangen wird, Bilder. Dies erlaubt der Bedienperson ein verbessertes Einschätzen der Gesamtsituation und auch der Empfehlung für die nächstfolgende Tätigkeit.

In einer vorteilhaften Weiterbildung im Rahmen der Prädiktion medizinischer Tätigkeiten kann vorgesehen sein, dass die Trainingsdatensätze wenigstens teilweise auch eine das klinische Ergebnis des Eingriffs, insbesondere bezogen auf die Gesundheit des Patienten, beschreibende Ergebnisinformation für jeweilige Eingriffe umfassen, wobei die Ausgangsdaten eine abgeschätzte und/oder referenzierte Prädiktionsinformation, die ein prädiziertes klinisches Ergebnis bei Durchführung der Tätigkeit beschreibt, umfassend ermittelt werden. Die Unterstützungsfunktion kann mithin dahingehend erweitert werden, dass sie auch erwartete klinische Ergebnisse ("clinical outcomes") beschreibt. So wird eine besser informierte klinische Entscheidung der Bedienperson erreicht. Mithin bilden insbesondere Ergebnisparameter Teil der Ausgangsdaten. In diesem Zusammenhang kann es auch besonders zweckmäßig sein, wenn wiederum auf weitere Eingriffe, die insbesondere den Trainingsdaten enthalten waren, referenziert wird. Eine vorteilhafte Weiterbildung kann zudem vorsehen, dass bei mehreren in den Ausgangsdaten vorgeschlagenen Tätigkeiten die Ausgangsdaten einen Vergleich der jeweiligen klinischen Ergebnisse umfassend ermittelt werden. Beispielsweise kann eine Ausgabe der Art "In Fällen mit ähnlichem Bildmaterial, bei denen eine weitere Spirale in dem Aneurysma platziert wurde, kam es zu einer Aneurysmaokklusion nach 6 Monaten. In Fällen, in denen keine Spirale in dem Aneurysma platziert wurde, kam es zu einer Aneurysmaokklusion nach 12 Monaten" entstehen. Hierzu können auch Bilder aus den referenzierten Fällen angezeigt werden. So entsteht für die verschiedenen empfohlenen Tätigkeiten auch eine Vergleichsgrundlage.

In einer besonders bevorzugten, zweiten Gruppe von Ausführungsbeispielen kann vorgesehen sein, dass für Ausgangsdaten, die unter Nutzung eines Eingangsdatensatzes für einen ersten der Zeitpunkte bestimmt werden und als empfohlene Tätigkeit eine technische Bedienhandlung an wenigstens einer der wenigstens einen Eingriffseinrichtung beschreiben, das Training derart durchgeführt wird, dass die in dem zugehörigen strukturierten Textdatensatz, insbesondere einer Log-Datei, für den dem ersten Zeitpunkt folgenden Zeitpunkt beschriebene Bedienhandlung als Grundwahrheit verwendet wird. Insbesondere sind Ausgestaltungen denkbar, in denen sich die Ausgangsdaten lediglich auf Bedienhandlungen an der wenigstens einen Eingriffseinrichtung beziehen. In diesem Fall wird also Systeminteraktionsunterstützung geboten. Beim Bezug auf Bedienhandlungen wird mit besonderem Vorteil ausgenutzt, dass sich diese in vielen Fällen bereits objektiv und automatisch aus den Trainingsdaten, insbesondere aus Log-Dateien, ergeben. Damit ist aber die Grundwahrheit beim Training bereits bekannt, denn letztlich muss zu dem dem ersten Zeitpunkt folgenden Zeitpunkt als Bedienhandlung das geschehen, was für diesen Zeitpunkt durch die Trainingsdaten, insbesondere die Log-Datei, beschrieben wird. Als symbolische Formel ausgedrückt muss also im Fall einer Log-Datei

$$\text{Log-Datei } [t_{i+1}] = \text{Unterstützungsfunktion}$$

(Eingangsdaten mit Log-Datei $[t_i]$) gelten, wobei $t_i$ dem ersten Zeitpunkt, $t_{i+1}$ dem nächstfolgenden Zeitpunkt entspricht. Die Unterstützungsfunktion lernt somit, zu einem ersten Zeitpunkt vorherzusagen, was Bedienpersonen zum dem ersten Zeitpunkt folgenden Zeitpunkt, insbesondere nächstfolgenden Zeitpunkt, für eine Interaktion mit der wenigstens einen technischen Eingriffseinrichtung durchführten.

Die Ausgangsdaten helfen in einem solchen Fall der Bedienperson, sich in den äußerst komplexen Bedienmöglichkeiten und Benutzerinterfaces von technischen Eingriffseinrichtungen, beispielsweise Angiographieeinrichtungen und/oder endovaskulären Robotern, zurechtzufinden. Dabei ist insbesondere eine Ausgabe von Ausgangsdaten bei der Nutzung der trainierten Unterstützungsfunktion zur Unterstützung der Bedienperson unmittelbar an den von der Bedienhandlung betroffenen technischen Eingriffseinrichtungen, insbesondere in deren Benutzerinterface, denkbar. So kann eine äußerst intuitive Unterstützung des Benutzers erfolgen. Der Bedienperson wird somit ideal assistiert. Sie kann von dem Wissen und der Erfahrung der Expertenpersonen profitieren und lernen. Es wird vorhergesagt, welche technische Bedienhandlung an der wenigstens einen technischen Eingriffseinrichtung die geeignetste wäre, was idealerweise rein unter Nutzung von ohnehin technisch aufgezeichneten Trainingsdaten für eine Mehrzahl von Eingriffen geschieht.

Insbesondere in einer Ausgestaltung, in der sich die Ausgangsdaten nur auf Bedienhandlungen beziehen sollen, mithin eine technische Assistenz zur Bedienung der Eingriffseinrichtungen gegeben wird, ergibt sich mithin ein computerimplementiertes Verfahren zum Bereitstellen einer trainierten Unterstützungsfunktion für einen bildüberwachten medizinischen Eingriff an einer Eingriffsanordnung, die wenigstens eine technische Eingriffseinrichtung aufweist, von der wenigstens eine eine medizinische Bildaufnahmeeinrichtung zur Bildüberwachung des Eingriffs ist, zur Unterstützung einer Bedienperson, umfassend folgende Schritte:

- Bereitstellen einer für mehrere Datentypen, umfassend zwei und/oder dreidimensionale Bilder und Videos und strukturierten Text, vortrainierten generativen Funktion, die aus Eingangsdaten der Datentypen auf die Eingangsdaten bezogene Ausgangsdaten generiert, als untrainierte Unterstützungsfunktion,

- Trainieren der Unterstützungsfunktion mit auf den bildüberwachten medizinischen Eingriff bezogenen Trainingsdatensätzen, wobei die Trainingsdatensätze für mehrere Zeitpunkte einer Eingriffsdauer jedes in den Trainingsdatensätzen beschriebenen Eingriffs wenigstens folgende Eingangsdaten für die Unterstützungsfunktion, die den Ablauf des Eingriffs bis zu dem jeweiligen Zeitpunkt beschreiben, enthalten

  o wenigstens einen Bilddatensatz der Bildüberwachung mit der Bildaufnahmeeinrichtung und
  o wenigstens einen strukturierten Textdatensatz, der wenigstens eine durchgeführte technische Bedienhandlung beschreibt und seitens wenigstens einer der wenigstens einen Eingriffseinrichtung vorliegt,

  und wobei das Training derart erfolgt, dass bei Trainingsdaten bis zu einem ersten Zeitpunkt enthaltenden Eingangsdaten die Ausgangsdaten zumindest eine empfohlene technische Bedienhandlung für die Bedienperson beschreiben und die empfohlene Bedienhandlung die Bedienhandlung, die in dem zugehörigen strukturierten Textdatensatz, insbesondere einer Log-Datei, für den dem ersten Zeitpunkt folgenden Zeitpunkt beschrieben ist, als Grundwahrheit umfasst, und

- Bereitstellen der trainierten Unterstützungsfunktion.

Wie bereits erwähnt, kann vorgesehen sein, dass die Ausgangsdaten auch einen Verweis auf wenigstens eine Referenzinformation enthalten. Konkret kann vorgesehen sein, dass die Ausgangsdaten Verweise auf Bild- und/oder Videomaterial, insbesondere der Trainingsdatensätze, das eine vergleichbare Eingriffssituation illustriert, umfassen. Auf diese Weise kann dem Benutzer auch bildlich verbessert illustriert werden, wie die empfohlene Tätigkeit zu verstehen ist und/oder durchzuführen ist. Insbesondere kann durch das Bild- und/oder Videomaterial gezeigt werden, wie eine Expertenperson der empfohlenen Vorgehensweise gefolgt ist.

Im Allgemeinen kann ferner zweckmäßig vorgesehen sein, dass wenigstens ein Teil der Trainingsdatensätze, der in einer letzten Trainingsphase zum Training verwendet wird, eine gemeinsame Eingriffsstrategie zur Durchführung des Eingriffs beschreibt, insbesondere eine in einer Arbeitsumgebung, in der die trainierte Unterstützungsfunktion angewendet werden soll, einzusetzende Eingriffsstrategie. So kann beispielsweise eine Behandlungsstrategie, die für eine Arbeitsumgebung, beispielsweise ein bestimmtes Krankenhaus, definiert wurde, als bevorzugt durch die trainierte Unterstützungsfunktion gelernt werden. Insbesondere können die Trainingsdaten für die letzte Trainingsphase dann Eingriffe aus der Arbeitsumgebung beschreiben, insbesondere solche Eingriffe, bei denen die Eingriffsstrategie befolgt wird. Insbesondere junge Kollegen können so die arbeitsumgebungsspezifischen Tätigkeiten bzw. deren arbeitsumgebungsspezifische Abfolge erlernen.

Für ein erfindungsgemäßes computerimplementiertes Verfahren zur Unterstützung einer Bedienperson bei einem bildüberwachten medizinischen Eingriff an einer Eingriffsanordnung, die wenigstens eine technische Eingriffseinrichtung aufweist, von der wenigstens eine eine medizinische Bildaufnahmeeinrichtung zur Bildüberwachung des Eingriffs ist, sind folgende Schritte vorgesehen:

- zu einem Zeitpunkt während des Eingriffs, Zusammenstellen eines Eingangsdatensatzes, der den Ablauf des Eingriffs bis zu dem Zeitpunkt beschreibt und zumindest Folgendes enthält:

    o wenigstens einen Bilddatensatz der Bildüberwachung mit der Bildaufnahmeeinrichtung, der von der Bildaufnahmeeinrichtung empfangen wird, und
    o wenigstens einen strukturierten Textdatensatz, der wenigstens eine durchgeführte technische Bedienhandlung und/oder wenigstens eine eingriffsbezogene, insbesondere bildgebungsbezogene, Eingriffsinformation beschreibt und von wenigstens einer der wenigstens einen Eingriffseinrichtung empfangen wird,

- Anwenden einer trainierten Unterstützungsfunktion, die insbesondere gemäß einem erfindungsgemäßen Bereitstellungsverfahren bereitgestellt wird, auf den Eingangsdatensatz zur Ermittlung von Ausgangsdaten, die zumindest eine empfohlene, als nächstes durchzuführende Tätigkeit der Bedienperson beschreiben, und
- Verwendung der Ausgangsdaten zur Unterstützung der Bedienperson.

Wie bereits erwähnt, gelten die Ausführungen für das erfindungsgemäße Bereitstellungsverfahren analog für das erfindungsgemäßen Bereitstellungsverfahren sowie die jeweils sonstigen Gegenstände fort. Insbesondere gilt das hinsichtlich der Eingangsdaten und der Ausgangsdaten der trainierten Unterstützungsfunktion Ausgeführte auch für das Bereitstellungsverfahren. Die trainierte Unterstützungsfunktion kann sich in Ausführungsbeispielen auch für das Bereitstellungsverfahren auf Bedienhandlungen an der wenigstens einen Eingriffseinrichtung beziehen oder beschränken.

Zur gezielten Unterstützung der Bedienperson mittels der Ausgangsdaten der trainierten Unterstützungsfunktion sind verschiedene konkrete Ansätze denkbar, insbesondere auch, was den Zeitpunkt der Unterstützung, insbesondere durch Ausgabe der Ausgangsdaten, angeht.

In einer ersten konkreten Möglichkeit kann für das Unterstützungsverfahren vorgesehen sein, dass die Ausgangsdaten auf eine mittels einer Benutzereingabe der Bedienperson ermittelte Anforderung ermittelt und an die Bedienperson ausgegeben werden. Beispielsweise kann die Bedienperson also, beispielsweise durch Betätigung eines Bedienelements, Spracheingabe und dergleichen die Unterstützung durch die trainierte Unterstützungsfunktion triggern, sodass die Ausgangsdaten ermittelt und an die Bedienperson ausgegeben werden. Besonders bevorzugt ist hierbei die Verwendung eines Spracheingabemittels, beispielsweise eines Mikrofons, welches auch für andere Sprachsteuerungsaufgaben im Rahmen der Eingriffsanordnung eingesetzt werden kann. Beispielsweise kann der Benutzer fragen: "System, was würde ein Experte nun tun?", um eine Anwendung der trainierten Unterstützungsfunktion und somit die konkrete Unterstützung durch Ausgabe von Ausgangsdaten zu triggern.

Dabei sei an dieser Stelle angemerkt, dass generell zur Ausgabe von Ausgangsdaten verschiedenste Ausgabemittel der Eingriffsanordnung, insbesondere der wenigstens einen Eingriffseinrichtung, eingesetzt werden können, um eine optische und/oder akustische, gegebenenfalls haptisch unterstützte, Ausgabe der Ausgabedaten an die Bedienperson zu erlauben. Insbesondere in Bezug auf technische Bedienhandlungen an den technischen Eingriffseinrichtungen ist es jedoch besonders bevorzugt, die Ausgabedaten wenigstens teilweise über eine entsprechende Benutzerschnittstelle der jeweiligen Eingriffseinrichtung auszugeben.

In einer zweiten, alternativ oder zusätzlich zur ersten Möglichkeit verwendbaren Möglichkeit kann mit besonderem Vorteil vorgesehen sein, dass

- die Ausgangsdaten wiederholt zu mehreren Zeitpunkten während des Eingriffs ermittelt werden,
- eine Abweichung einer aktuellen, insbesondere noch nicht abgeschlossenen und/oder noch nicht wirkenden, Tätigkeit der Bedienperson von der gemäß den Ausgangsdaten empfohlenen Tätigkeit gemäß einer Metrik ermittelt wird, und
- bei Überschreitung eines Schwellwerts für die Abweichung die Ausgangsdaten, insbesondere unter Bezug auf und/oder Vergleich mit der aktuellen Tätigkeit, an die Bedienperson als Empfehlung ausgegeben werden.

In dieser Ausgestaltung ist die trainierte Unterstützungsfunktion überwachend tätig. Ausgangsdaten werden wiederholt während des Eingriffs ermittelt, insbesondere immer dann, wenn eine neue Tätigkeit im Sinne der Ausgangsdaten vorliegt bzw. beginnt, um die tatsächlich durchgeführte Tätigkeit mit der aktuellen tatsächlichen Tätigkeit der Bedienperson vergleichen zu können. Um diesen Vergleich zu erlauben, kann beispielsweise eine Metrik verwendet werden, die die Abweichung quantitativ beschreibt. Ist die Abweichung zu stark, kann die Bedienperson durch entsprechende Ausgabe hierauf hingewiesen werden und es kann die gemäß der Ausgangsdaten empfohlene Tätigkeit empfohlen werden. Dies ist insbesondere dann zweckmäßig, wenn die aktuelle tatsächliche Tätigkeit noch nicht abgeschlossen ist oder aber ihre Wirkung noch nicht entfaltet hat. Dies ist bei Bedienhandlungen an der wenigstens einen Eingriffseinrichtung häufig der Fall, da in vielen Fällen zunächst eine Eingabe erfolgt, die erst danach tatsächlich durch Aktorik umgesetzt wird. Bevor nun die Aktorik angesteuert wird, findet der Vergleich statt, um gegebenenfalls auf bessere Optionen hinweisen zu können.

Konkret kann beispielsweise vorgesehen sein, dass bei einer übereinstimmenden oder einer gleichen Klasse von Tätigkeiten angehörender aktueller Tätigkeit und empfohlener Tätigkeit die Abweichung als eine Differenz einer Wirkungsstärke der jeweiligen Tätigkeiten ermittelt wird. Betrifft die Tätigkeit beispielsweise die Verstellung der Aufnahmeanordnung der Bildaufnahmeeinrichtung, kann die Stärke der Verstellung für die aktuelle Tätigkeit der Bedienperson und die empfohlene Tätigkeit verglichen werden, um die Abweichung entsprechend zu bestimmen. Konkrete Anwendungsbeispiele für eine solche Metrik umfassen beispielsweise die Verstellung eines C-Bogens um einen bestimmten Winkel, den Vorschub eines medizinischen Instruments mit einem endovaskulären Roboter um eine bestimmte Strecke, eine Anpassung einer Röntgendosis auf einen bestimmten Wert und dergleichen.

In allen Fällen können die Ausgangsdaten hier um Informationen zur Abweichung bzw. zur aktuellen Tätigkeit ergänzt werden. Eine Ausgabe kann beispielsweise lauten: "Wird der C-Bogens um 5° weiter gedreht als geplant, können in der aktuellen Eingriffssituation den Eingriffsbereich besser zeigende Fluoroskopiebilder erhalten werden."

Wie bezüglich des Bereitstellungsverfahrens bereits diskutiert, können Ausgangsdaten einen Verweis auf Referenzinformationen enthalten, beispielsweise auf Bild- und/oder Videomaterial. Dann kann vorgesehen sein, dass, bei einen Verweis enthaltenden, auszugebenden Ausgangsdaten wenigstens ein Teil des Verweisziels mit ausgegeben wird. Beispielsweise können entsprechend Bilder vergleichbarer Eingriffssituationen und/oder Bediensituationen ausgegeben werden und/oder erwartete klinische Ergebnisse anhand von Referenzfällen verglichen werden.

Eine erfindungsgemäße Bereitstellungseinrichtung zum Bereitstellen einer trainierten Unterstützungsfunktion für einen bildüberwachten medizinischen Eingriff an einer Eingriffsanordnung, die wenigstens eine technische Eingriffseinrichtung aufweist, von der wenigstens eine eine medizinische Bildaufnahmeeinrichtung zur Bildüberwachung des Eingriffs ist, zur Unterstützung einer Bedienperson, weist auf:

- eine erste Bereitstellungsschnittstelle zum Empfang einer für mehrere Datentypen, umfassend zwei und/oder dreidimensionale Bilder und Videos und strukturierten Text, vortrainierten generativen Funktion, die aus Eingangsdaten der Datentypen auf die Eingangsdaten bezogene Ausgangsdaten generiert, als untrainierte Unterstützungsfunktion,
- eine zweite Bereitstellungsschnittstelle zum Empfang von Trainingsdatensätzen, wobei die Trainingsdatensätze für mehrere Zeitpunkte einer Eingriffsdauer jedes in den Trainingsdatensätzen beschriebenen Eingriffs wenigstens folgende Eingangsdaten für die Unterstützungsfunktion, die den Ablauf des Eingriffs bis zu dem jeweiligen Zeitpunkt beschreiben, enthalten:

    o wenigstens einen Bilddatensatz der Bildüberwachung mit der Bildaufnahmeeinrichtung und
    o wenigstens einen strukturierten Textdatensatz, der wenigstens eine durchgeführte technische Bedienhandlung und/oder wenigstens eine eingriffsbezogene Eingriffsinformation beschreibt und seitens wenigstens einer der wenigstens einen Eingriffseinrichtung vorliegt,

- eine Trainingseinheit zum Trainieren der Unterstützungsfunktion mit den Trainingsdatensätzen, wobei das Training derart erfolgt, dass bei Trainingsdaten bis zu einem Zeitpunkt enthaltenden Eingangsdaten die Ausgangsdaten zumindest eine empfohlene Tätigkeit für die Bedienperson beschreiben und die empfohlene Tätigkeit die nächstfolgende Tätigkeit der Bedienperson gemäß den Trainingsdaten und/oder eine am besten bewertete Tätigkeit bei Einsatz von bestärkendem Lernen umfasst, und
- eine dritte Bereitstellungsschnittstelle zum Bereitstellen der trainierten Unterstützungsfunktion.

Die Bereitstellungseinrichtung ist mithin zur Durchführung eines erfindungsgemäßen Bereitstellungsverfahrens ausgebildet. Sie weist wenigstens einen Prozessor und wenigstens ein Speichermittel auf. Durch Hardware und/oder Software werden Funktionseinheiten gebildet, um Schritte des erfindungsgemäßen Bereitstellungsverfahrens durchzuführen. Neben den genannten Funktionseinheiten können auch weitere, vorteilhafte Ausgestaltungen des Bereitstellungsverfahrens betreffende Funktionseinheiten vorgesehen werden, beispielsweise eine Ergänzungseinheit, die den Trainingsdaten Experteninformation hinzufügt, und dergleichen.

Ein erfindungsgemäßes Bereitstellungscomputerprogramm ist direkt in ein Speichermittel einer Bereitstellungseinrichtung ladbar und weist Programmmittel derart auf, dass bei Ausführung des Computerprogramms auf der Bereitstellungseinrichtung diese veranlasst wird, die Schritte eines erfindungsgemäßen Bereitstellungsverfahrens durchzuführen. Das Bereitstellungscomputerprogramm kann auf einem erfindungsgemäßen, elektronisch lesbaren Datenträger gespeichert sein, welcher mithin darauf gespeicherte Steuerinformationen aufweist, die wenigstens ein erfindungsgemäßes Bereitstellungscomputerprogramm umfassen und derart ausgestaltet sind, dass bei Verwendung des Datenträgers in einer Bereitstellungseinrichtung diese ausgebildet wird, ein erfindungsgemäßes Bereitstellungsverfahren auszuführen.

Eine erfindungsgemäße Steuereinrichtung zur Unterstützung einer Bedienperson bei einem bildüberwachten medizinischen Eingriff an einer Eingriffsanordnung, die die Steuereinrichtung und wenigstens eine technische Eingriffseinrichtung aufweist, von der wenigstens eine eine medizinische Bildaufnahmeeinrichtung zur Bildüberwachung des Eingriffs ist, umfasst:

- eine erste Anwendungsschnittstelle zum Empfang von wenigstens einem Bilddatensatz der Bildüberwachung von der Bildaufnahmeeinrichtung und wenigstens einem strukturierten Textdatensatz, der wenigstens eine durchgeführte technische Bedienhandlung und/oder wenigstens eine eingriffsbezogene Eingriffsinformation beschreibt, von wenigstens einer der wenigstens einen Eingriffseinrichtung,
- eine Zusammenstellungseinheit zum Zusammenstellen eines Eingangsdatensatzes zu einem Zeitpunkt während des Eingriffs, wobei der Eingangsdatensatz den Ablauf des Eingriffs bis zu dem Zeitpunkt beschreibt und den wenigstens einen Bilddatensatz und den wenigstens einen strukturierten Textdatensatz enthält,
- eine Anwendungseinheit zum Anwenden einer trainierten Unterstützungsfunktion auf den Eingangsdatensatz zur Ermittlung von Ausgangsdaten, die zumindest die zumindest eine empfohlene, als nächstes durchzuführende Tätigkeit der Bedienperson beschreiben, und
- eine Unterstützungseinheit zur Verwendung der Ausgangsdaten zur Unterstützung der Bedienperson.

Die Steuereinrichtung ist mithin zur Durchführung eines erfindungsgemäßen Unterstützungsverfahrens ausgebildet. Sie weist wenigstens einen Prozessor und wenigstens ein Speichermittel auf. Durch Hardware und/oder Software werden Funktionseinheiten gebildet, um Schritte des erfindungsgemäßen Unterstützungsverfahrens durchzuführen. Neben den genannten Funktionseinheiten können auch weitere, vorteilhafte Ausgestaltungen des Unterstützungsverfahrens betreffende Funktionseinheiten vorgesehen werden, beispielsweise eine Interaktionseinheit zur Ermittlung einer Anforderung aus einer Benutzereingabe und/oder zur Ausgabe von Ausgangsdaten und/oder Referenzinformationen, eine Triggereinheit zur Ermittlung der Abweichung und zur Durchführung des Vergleichs und dergleichen.

Die Erfindung betrifft auch eine Eingriffsanordnung, aufweisend eine erfindungsgemäße Steuereinrichtung und wenigstens eine technische Eingriffseinrichtung, von der wenigstens eine eine medizinische Bildaufnahmeeinrichtung zur Bildüberwachung des Eingriffs ist. Die Bildaufnahmeeinrichtung kann insbesondere eine Angiographieeinrichtung und/oder eine Röntgeneinrichtung mit einem C-Bogen sein. Eine weitere Eingriffseinrichtung kann ein endovaskulärer Roboter sein. Die Steuereinrichtung kann eine zentrale Steuerinstanz der Eingriffsanordnung sein. Sie kann dabei in eine der Eingriffseinrichtungen integriert und/oder dieser zugeordnet sein und/oder steuernden Zugriff auf die Benutzerschnittstelle der wenigstens einen Eingriffseinrichtung haben. Allgemein gesagt kann die Eingriffsanordnung, insbesondere wenigstens ein Ausgabemittel, insbesondere als Teil wenigstens einer der wenigstens einen Eingriffseinrichtung, aufweisen. Das Ausgabemittel kann zur Ausgabe von Bilddaten und/oder Ausgabedaten bzw. daraus abgeleiteten Daten genutzt werden.

Ein erfindungsgemäßes Unterstützungscomputerprogramm ist direkt in ein Speichermittel einer Steuereinrichtung ladbar und weist Programmmittel derart auf, dass bei Ausführung des Computerprogramms auf der Steuereinrichtung diese veranlasst wird, die Schritte eines erfindungsgemäßen Unterstützungsverfahrens durchzuführen. Das Unterstützungscomputerprogramm kann auf einem erfindungsgemäßen, elektronisch lesbaren Datenträger gespeichert sein, welcher mithin darauf gespeicherte Steuerinformationen aufweist, die wenigstens ein erfindungsgemäßes Unterstützungscomputerprogramm umfassen und derart ausgestaltet sind, dass bei Verwendung des Datenträgers in einer Steuereinrichtung diese ausgebildet wird, ein erfindungsgemäßes Unterstützungsverfahren auszuführen.

Weitere Vorteile und Einzelheiten der vorliegenden Erfindung ergeben sich aus den im Folgenden beschriebenen Ausführungsbeispielen sowie anhand der Zeichnungen. Dabei zeigen:

Fig. 1    eine graphische Erläuterung zu einer generativen vortrainierten Funktion mit multimodalen Eingangsdaten,

Fig. 2    eine Prinzipskizze zur Erläuterung eines ersten Ausführungsbeispiels des erfindungsgemäßen Bereitstellungsverfahrens,

Fig. 3    eine Prinzipskizze zur Erläuterung eines zweiten Ausführungsbeispiels des erfindungsgemäßen Bereitstellungsverfahrens,

Fig. 4    einen Ablaufplan eines allgemeinen Ausführungsbeispiels eines erfindungsgemäßen Unterstützungsverfahrens,

Fig. 5    die funktionale Struktur einer erfindungsgemäßen Bereitstellungseinrichtung,

Fig. 6    schematisch eine erfindungsgemäße Eingriffsanordnung, und

Fig. 7    die funktionale Struktur einer Steuereinrichtung der Eingriffsanordnung.

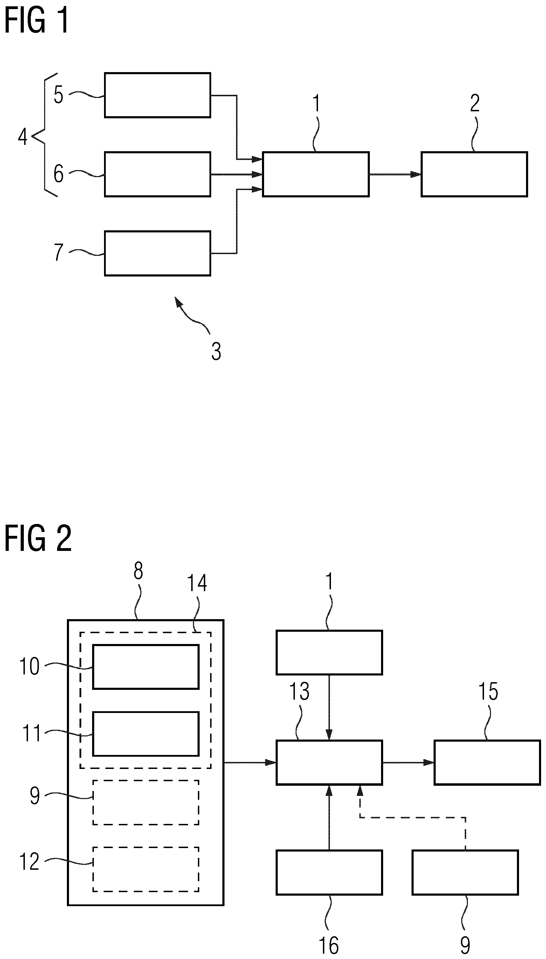

Fig. 1 erläutert schematisch eine vortrainierte generative Funktion 1, wie sie die Grundlage der folgenden Ausführungsbeispiele bietet. Bei der vortrainierten generativen Funktion 1 handelt es sich vorliegend um ein Large Language Model (LLM), dessen Ausgangsdaten 2 generierten Text umfassen. Die vortrainierte generative Funktion 1 ist multimodal, da ihre Eingangsdaten 3, auf die sie trainiert wurde, unterschiedliche Arten von Bilddaten 4, nämlich zweidimensionale und dreidimensionale Bilddaten 5 sowie Videodaten 6 (also insbesondere eine Zeitserie von Bildern), und auch strukturierte Textdaten 7 umfassen.

Die im Folgenden dargestellten Ausführungsbeispiele zeichnen sich allgemein dadurch aus, dass sie eine Bedienperson bei einem medizinischen Eingriff, insbesondere einem minimalinvasiven Eingriff mit wenigstens einem medizinischen Instrument, unterstützen sollen. Hierzu wird vorliegend eine Eingriffsanordnung verwendet, die mehrere Eingriffseinrichtungen umfasst. Eine erste Eingriffseinrichtung ist eine Bildaufnahmeeinrichtung, die zur Aufnahme von prä- und/oder postinterventionellen Bilddatensätzen eingesetzt werden kann, in jedem Fall aber zur Bildüberwachung des Eingriffs eingesetzt wird. In den hier beschriebenen Ausführungsbeispielen wird eine Angiographieeinrichtung bzw. Röntgeneinrichtung mit einem C-Bogen verwendet, die zur Bildüberwachung beispielsweise Fluoroskopiebilder (als weitere Bilddatensätze) aufnimmt, beispielsweise als eine Art "Video". In anderen Ausführungsbeispielen sind auch andere Bildaufnahmeeinrichtungen, beispielsweise CT- und/oder Magnetresonanzeinrichtungen, denkbar.

Als weitere Eingriffseinrichtung wird vorliegend beispielhaft ein endovaskulärer Roboter verwendet, der die Bewegung des medizinischen Instruments in dem Patienten unterstützt.

Die betrachteten Eingriffe beziehen sich auf eine bestimmte Eingriffsart, beispielsweise Aneurysmabehandlungen mit Spiralen (Coiling), Embolisationen und dergleichen.

Für zuvor durchgeführte Eingriffe werden in allen hier beschriebenen Fällen Bilddatensätze des Eingriffs, die mit der Bildaufnahmeeinrichtung aufgenommen wurden, und strukturierte Textdatensätze, vorliegend wenigstens Log-Files der Eingriffseinrichtungen, die Bedienhandlungen und eingestellte Betriebsparameter beschreiben, gespeichert. Optionale weitere, technisch automatisch erfassbare und zu speichernde (und später als Trainingsdaten zu verwendende) Daten umfassen Vorab-Bilddatensätze und Konfigurationsdateien oder Teile davon, beispielsweise DICOM-Metadatensätze.

Fig. 2 zeigt eine Prinzipskizze eines ersten Ausführungsbeispiels des erfindungsgemäßen Bereitstellungsverfahrens, nämlich der ersten Gruppe, in der eine Unterstützungsfunktion bereitgestellt werden soll, die die Bedienperson hinsichtlich des medizinischen, auf den Eingriff bezogenen Vorgehens unterstützen soll, insbesondere mit Untersuchungs- und Behandlungsoptionen. Anders gesagt soll wenigstens eine medizinische Tätigkeit, die als nächstes durchzuführen ist, empfohlen werden.

Hierzu werden Trainingsdatensätze 8 vergangener Eingriffe bereitgestellt, wobei die Eingriffe bevorzugt von erfahrenen Bedienpersonen (Experten-Bedienpersonen) durchgeführt wurden. Deren Wissen kann als Experteninformation 9 jedoch auch anderweitig bereitgestellt werden, worauf im Folgenden noch genauer eingegangen werden wird. Die Trainingsdatensätze 8 umfassen für jeden Eingriff für dessen gesamte Eingriffsdauer Bilddatensätze 10 und strukturierte Textdatensätze 11. Optional können die Trainingsdatensätze 8 für die Eingriffe auch eine Ergebnisinformation 12 für jeden Eingriff enthalten, die das klinische Ergebnis beschreiben.

Neben den Trainingsdatensätzen 8 wird die vortrainierte generative Funktion 1 als untrainierte Unterstützungsfunktion bereitgestellt, die domänenspezifisch trainiert werden soll. Das Training geschieht in einem Schritt 13, derart, dass die trainierte Unterstützungsfunktion 15, wenn sie mit den technisch verfügbaren Eingangsdaten 14, umfassend die Bilddatensätze 10 (2D-/3D-Bilddatensätze, Fluoroskopiebilderserie) und die strukturierten Textdatensätze 11 (Log-Files) bis zu einem Zeitpunkt während des jeweiligen Eingriffs, als Eingangsdatensatz versorgt wird, Ausgangsdaten ausgibt, die die medizinische Tätigkeit beschreiben, die die Experten-Bedienperson gemäß den Trainingsdaten 8 oder anderweitig zugeführter Experteninformationen zum nächstfolgenden Zeitpunkt des Eingriffs vorgenommen hat oder vornehmen würde. Allein diese technisch automatisch verfügbaren und multimodalen Eingangsdaten 14 werden auch in der Anwendung (Inferenz) der trainierten Unterstützungsfunktion 15 angewendet.

Die medizinische Tätigkeit zum nächstfolgenden Zeitpunkt kann als Grundwahrheit aus Bilddatensätzen 10 und strukturierten Textdatensätzen 11 zu späteren Zeitpunkten abgeleitet werden, aus mit den Trainingsdatensätzen 8 geliefertem Expertenwissen (Experteninformation 9), beispielsweise Protokollen oder Annotationen, oder nachträglich durch Annotation bereitgestellt werden. Dabei muss das Expertenwissen nicht vollständig von einem menschlichen Experten stammen, denn es existieren drei Optionen, die auch ergänzend eingesetzt werden können. Alle Optionen dienen dazu, das medizinische Verständnis der Eingriffssituation, wie sie durch die Eingangsdaten 14 für den betrachteten Zeitpunkt beschrieben wird, zu etablieren.

In einer ersten Option annotiert ein menschlicher Experte die Eingangsdaten 14 mit Expertenwissen 9, insbesondere basierend auf den Bilddatensätzen 10.

In einer zweiten Option wird ausgenutzt, dass bereits Vorarbeit zur Interpretation von Eingriffssituationen auf der Basis von Bilddatensätzen geleistet ist, indem eine trainierte Expertisefunktion auf die Bilddatensätze 10 angewendet wird, um das Expertenwissen 9 wenigstens teilweise zu generieren. In einer dritten Option wird bestärkendes Lernen eingesetzt, um das Expertenwissen 9 als Bewertung in den Trainingsprozess des Schrittes 13 angehen zu lassen.

Als "guard rails" werden bereits im Rahmen der Bereitstellung zudem Ausschlussbedingungen 16 verwendet, um bestimmte Empfehlungen zu vermeiden. Auf diese Weise kann vermieden werden, dass ungewollte Interaktionen bzw. allgemein Tätigkeiten, die beispielsweise Risiken für den Patienten mit sich bringen, allgemein unsinnig sind oder für die technischen Eingriffseinrichtungen und/oder deren Nutzung nachteilhaft sind, als Empfehlung verhindert werden können. Ausschlussbedingungen können für Eingangs- und/oder Ausgangsdaten spezifisch sein (beispielsweise keine zu hohen Röntgendosen, keine zu langen Bewegungswege, keine Bewegung aus dem Eingriffsbereich und dergleichen), aber auch allgemein gültig sein (beispielsweise kein Herunterfahren einer technischen Eingriffseinrichtung und/oder einer die trainierte Unterstützungsfunktion durchführenden Steuereinrichtung, grundsätzlich als Kollisionsschutz nicht einzunehmende Stellungen einer Eingriffseinrichtung). Ausschlussbedingungen 16 betreffen im Allgemeinen also solche Tätigkeiten, die eine Experten-Bedienperson nicht durchführen würde.

Die Ausschlussbedingungen 16 werden vorliegend in das Training selbst eingebracht, was vorliegend auf zwei Arten geschehen kann. Zunächst können sie unmittelbar eingehen, beispielsweise als nutzerseitige oder anderweitige Bewertung im bestärkenden Lernen. Denkbar ist es aber auch, dass die Ausschlussbedingungen 16 in einer zusätzlichen Überprüfung der Ausgangsdaten durch eine Überprüfungsfunktion realisiert werden. Die Überprüfungsfunktion kann in Ausführungsbeispielen selbst ein maschinenlehrbares Modell sein und kann gemeinsam mit der Unterstützungsfunktion trainiert werden. Möglich ist aber auch eine einfachere Umsetzung der Überprüfungsfunktion, beispielsweise als Liste auszuschließender Tätigkeiten.

Im Schritt 13 wird die Unterstützungsfunktion vorliegend ferner so trainiert, dass die Ausgangsdaten Zusatzinformationen enthalten, die weiter unterstützend wirken. So kann zum einen ein Verweis auf Referenzinformation enthalten sein, die mit den Ausgangsdaten ausgegeben werden können. Dies können beispielsweise Bilder ähnlicher Eingriffssituationen sein, auch solche, die sozusagen die Zukunft zeigen, also die Eingriffssituation, wenn dem empfohlenen Pfad gefolgt wird. Diese Referenzinformationen können in den Trainingsdaten enthalten sein und später in einer Datenbank verfügbar gehalten werden. Darüber hinaus können die Ausgangsdaten eine Prädiktionsinformation enthalten, die unter Nutzung der Ergebnisinformation 12 während des Trainings ein prädiziertes klinisches Ergebnis bei Durchführung der empfohlenen Tätigkeit beschreibt. Auch hierbei kann eine Referenz und, bei mehreren in den Ausgangsdaten empfohlenen Tätigkeiten, ein Vergleich erfolgen. Insgesamt können sich beispielsweise Ausgangsdaten ergeben, die Heilungschancen bei verschiedenen Vorgehensweisen beschreiben und dies noch mit Bildern verdeutlichen.

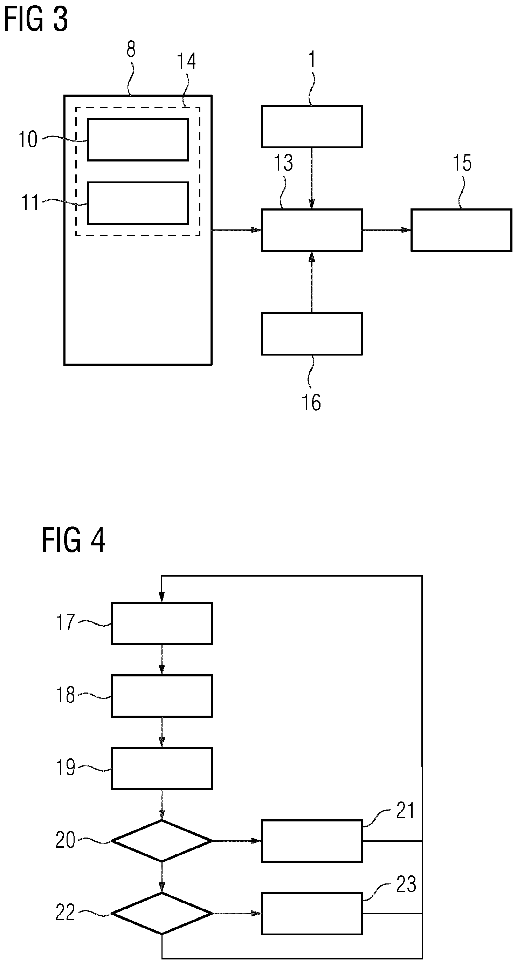

Fig. 3 zeigt ein Ausführungsbeispiel einer zweiten Gruppe, vorliegend einen Fall, in dem die resultierende Unterstützungsfunktion nur bei der Bedienung der technischen Eingriffseinrichtungen assistieren soll ("system interaction support"). Der Einfachheit halber sind gleiche Objekte mit gleichen Bezugszeichen bezeichnet.

Im Unterschied zu dem ersten Ausführungsbeispiel umfassen die Trainingsdatensätze 8 hier nur die Bilddatensätze 10 und die strukturierten Textdatensätze 11, da in diesen alle relevanten Informationen enthalten sind. Denn die Trainingsdatensätze 8 betreffen abgeschlossene, vergangene, insbesondere von Experten durchgeführte Eingriffe, in denen alle technischen Bedienhandlungen an den Eingriffseinrichtungen in den strukturierten Textdatensätzen 11 über die ganze Zeitdauer enthalten sind, das heißt, zu jedem Zeitpunkt innerhalb des Eingriffs sind sowohl die Eingangsdaten 14 bis dahin als auch die nächstfolgende Bedienhandlung bekannt, da diese ja automatisch protokolliert sind. Das bedeutet, die Grundwahrheit und die Eingangsdaten 14 für die Zeitpunkte für das Training im Schritt 13 liegen vor. Die Experteninformation 9 wird nicht benötigt. Als Pseudoformel für eine Log-Datei als strukturierter Textdatensatz 11 ausgedrückt erfolgt das Training also so, dass

$$\text{Log-Datei } [t_{i+1}] = \text{Unterstützungsfunktion}$$

(Eingangsdaten 14 mit Log-Datei $[t_i]$) gelten, wobei $t_i$ dem ersten Zeitpunkt, $t_{i+1}$ dem nächstfolgenden Zeitpunkt entspricht.

Auch hier werden Ausschlussbedingungen 16 umgesetzt, um beispielsweise zu starke Veränderungen zu vermeiden, Grenzwerte einzuhalten und dergleichen.

Beide dargestellten Ausführungsbeispiele des Bereitstellungsverfahrens haben gemein, dass zumindest in einer letzten Trainingsphase Trainingsdatensätze 8 zum Training verwendet werden, die eine in einer Arbeitsumgebung, in der die trainierte Unterstützungsfunktion 15 angewendet werden soll, einzusetzende Eingriffsstrategie beschreiben. Das Training erfolgt also so, dass die empfohlenen Tätigkeiten einer bestimmten Strategie zuträglich sind, die in der Arbeitsumgebung, beispielsweise einer Klinik, umgesetzt werden soll.

Fig. 4 zeigt einen Ablaufplan eines allgemeinen Ausführungsbeispiels des erfindungsgemäßen Unterstützungsverfahrens, wie es sowohl für die in dem ersten Ausführungsbeispiel als auch in dem zweiten Ausführungsbeispiel des Bereitstellungsverfahrens bereitgestellte trainierte Unterstützungsfunktion 15 durchgeführt werden kann. Dabei werden während eines Eingriffs in einem Schritt 17 Bilddatensätze 10 und strukturierte Textdatensätze 11, hier wiederum Log-Dateien, von den Eingriffseinrichtungen empfangen, die bis zum aktuellen Zeitpunkt des Eingriffs das gesamte Bildmaterial und Log-Material des Eingriffs umfassen, also den gesamten

Ablauf des Eingriffs bis zu dem aktuellen Zeitpunkt beschreiben. Diese werden in einem Schritt 18 zu den Eingangsdaten 14 des aktuellen Zeitpunkts zusammengestellt. In einem Schritt 19 wird dann die trainierte Unterstützungsfunktion 15 auf den zusammengestellten Eingangsdatensatz angewendet, um Ausgangsdaten mit der wenigstens einen empfohlenen Tätigkeit zu ermitteln.

In einem Schritt 20 wird dann überprüft, ob eine aus einer Benutzereingabe folgende Anforderung vorliegt. Beispielsweise kann diese aus einer sprachlichen Benutzereingabe über ein Mikrofon der Eingriffsanordnung folgen, aber auch aus einer Betätigung eines Bedienelements oder dergleichen. Liegt die Anforderung vor, werden in einem Schritt 21 über entsprechende Ausgabemittel die Ausgangsdaten ausgegeben, wobei insbesondere bei einer mit dem zweiten Ausführungsbeispiel bereitgestellten trainierten Unterstützungsfunktion 15 die Benutzerschnittstellen der Eingriffseinrichtungen, auf die sich die empfohlene Bedienhandlung bezieht, genutzt werden.

In einem Schritt 22 wird, wenn keine Anforderung vorliegt, eine Abweichung einer aktuell durchgeführten Tätigkeit der zu unterstützenden Bedienperson von der empfohlenen Tätigkeit, beispielsweise mittels einer Metrik, bestimmt und mit einem Grenzwert verglichen. Ist dieser überschritten, erfolgt in einem Schritt 23 wiederum die Ausgabe der Ausgangsdaten, in diesem Fall gegebenenfalls der aktuell durchgeführten Tätigkeit gegenübergestellt. Sowohl im Schritt 23 als auch im Schritt 21 können, falls vorgesehen, auch Referenzinformationen genutzt werden. Bei einer übereinstimmenden oder einer gleichen Klasse von Tätigkeiten angehörenden aktuellen Tätigkeit und empfohlenen Tätigkeit kann die Metrik als eine Differenz einer Wirkungsstärke der jeweiligen Tätigkeiten ermittelt werden.

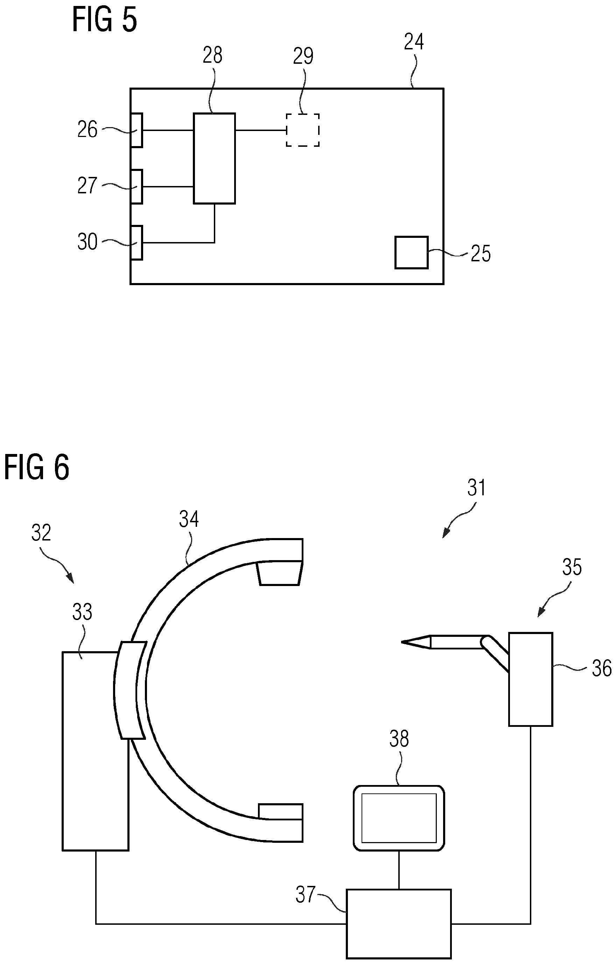

Fig. 5 zeigt eine funktionale Prinzipskizze einer erfindungsgemäßen Bereitstellungseinrichtung 24 zur Bereitstellung der trainierten Unterstützungsfunktion 15. Diese weist neben einem Speichermittel 25 eine erste Bereitstellungsschnittstelle 26 zum Empfang der vortrainierten generativen Funktion 1 und eine zweite Bereitstellungsschnittstelle 27 zum Empfang von Trainingsdatensätzen 8 auf. Eine Trainingseinheit 28 ist zum Trainieren der Unterstützungsfunktion gemäß Schritt 13 vorgesehen. Optional kann eine Ergänzungseinheit 29 zur Ergänzung von Experteninformationen 9 vorhanden sein. Mittels einer dritten Bereitstellungsschnittstelle 30 kann die trainierte Unterstützungsfunktion 15 bereitgestellt werden.

Fig. 6 zeigt schematisch eine erfindungsgemäße Eingriffsanordnung 31. Diese umfasst als eine erste Eingriffseinrichtung 32 eine Bildaufnahmeeinrichtung 33, hier eine Röntgeneinrichtung mit einem C-Bogen 34, konkret eine Angiographieeinrichtung. Als zweite Eingriffseinrichtung 35 ist ein endovaskulärer Roboter 36 vorgesehen, der die Bewegung eines medizinischen Instruments unterstützt. Der Betrieb der Eingriffsanordnung 31 wird von einer Steuereinrichtung 37 gesteuert, die zur Durchführung des erfindungsgemäßen Unterstützungsverfahrens ausgebildet ist. Die Steuereinrichtung 37 kann beiden Eingriffseinrichtungen 32, 35 zugeordnet sein und die Bilddatensätze 10 und die strukturierten Textdatensätze 11 über interne Schnittstellen bereitstellen. Auch eigene Steuereinheiten für die Eingriffseinrichtungen 32, 35 sind denkbar. Auch Ausgabemittel 38 können für beide Eingriffseinrichtungen 32, 35 getrennt und/oder gemeinsam vorgesehen werden.

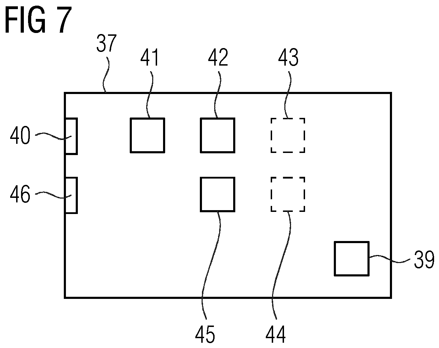

Fig. 7 zeigt den funktionalen Aufbau der Steuereinrichtung 37 genauer. Diese weist ein Speichermittel 39 auf. Mittels einer ersten Anwendungsschnittstelle 40 können gemäß Schritt 17 Bilddatensätze 10 und strukturierte Textdatensätze 11 empfangen werden. Die Steuereinrichtung 37 weist ferner eine Zusammenstellungseinheit 41 zum Zusammenstellen des Eingangsdatensatzes zu einem aktuellen Zeitpunkt während des Eingriffs gemäß Schritt 18 und eine Anwendungseinheit 42 zum Anwenden der trainierten Unterstützungsfunktion 15 auf den Eingangsdatensatz gemäß Schritt 19 auf.

Eine optionale Interaktionseinheit 43, die Benutzerschnittstellen der Eingriffseinrichtungen 32, 35 nutzen kann, kann zur Ermittlung der Anforderung aus einer Benutzereingabe gemäß Schritt 20 dienen. Entsprechend optional kann eine Triggereinheit 44 zur Ermittlung der Abweichung und zum Vergleich und ggf. Triggern der Ausgabe gemäß Schritt 22 vorgesehen sein.

Eine Unterstützungseinheit 45 dient zur Ausgabe der Ausgangsdaten, mithin der Empfehlung, an die Bedienperson. Hierzu können entsprechend die Ausgangsdaten an die Ausgabemittel 38 über eine zweite Anwendungsschnittstelle 46 weitergegeben werden.

Auch weitere Anwendungsschnittstellen können vorgesehen sein, beispielsweise zum Abruf von Referenzinformationen, auf die Ausgangsdaten verweisen, aus einer Datenbank, die beispielsweise herstellerseitig oder anderweitig auf einem Cloud-Server bereitgestellt werden kann.

Es sei abschließend noch angemerkt, dass selbstverständlich auch bei Eingriffen, bei denen die trainierte Unterstützungsfunktion 15 angewendet wird, neue Trainingsdatensätze 8 zum weiteren Training aufgezeichnet und verwendet werden können.

Unabhängig vom grammatikalischen Geschlecht eines bestimmten Begriffes sind Personen mit männlicher, weiblicher oder anderer Geschlechteridentität mit umfasst.

## Patentansprüche

1. Computerimplementiertes Verfahren zum Bereitstellen einer trainierten Unterstützungsfunktion (15) für einen bildüberwachten medizinischen Eingriff an einer Eingriffsanordnung (31), die wenigstens eine technische Eingriffseinrichtung (32, 35) aufweist, von der wenigstens eine eine medizinische Bildaufnahmeeinrichtung (33) zur Bildüberwachung des Eingriffs ist, zur Unterstützung einer Bedienperson, umfassend folgende Schritte:

   - Bereitstellen einer für mehrere Datentypen, umfassend zwei und/oder dreidimensionale Bilder und Videos und strukturierten Text, vortrainierten generativen Funktion (1), die aus Eingangsdaten (3) der Datentypen auf die Eingangsdaten (3) bezogene Ausgangsdaten (2) generiert, als untrainierte Unterstützungsfunktion,
   - Trainieren der Unterstützungsfunktion mit auf den bildüberwachten medizinischen Eingriff bezogenen Trainingsdatensätzen (8), wobei die Trainingsdatensätze (8) für mehrere Zeitpunkte einer Eingriffsdauer jedes in den Trainingsdatensätzen (8) beschriebenen Eingriffs wenigstens folgende Eingangsdaten (14) für die Unterstützungsfunktion, die den Ablauf des Eingriffs bis zu dem jeweiligen Zeitpunkt beschreiben, enthalten

       o wenigstens einen Bilddatensatz (10) der Bildüberwachung mit der Bildaufnahmeeinrichtung (33) und
       o wenigstens einen strukturierten Textdatensatz (11), der wenigstens eine durchgeführte technische Bedienhandlung und/oder wenigstens eine eingriffsbezogene Eingriffsinformation beschreibt und seitens wenigstens einer der wenigstens einen Eingriffseinrichtung (32, 35) vorliegt,

   und wobei das Training derart erfolgt, dass bei Trainingsdaten bis zu einem Zeitpunkt enthaltenden Eingangsdaten (14) die Ausgangsdaten zumindest eine empfohlene Tätigkeit für die Bedienperson beschreiben und die empfohlene Tätigkeit die nächstfolgende Tätigkeit der Bedienperson gemäß den Trainingsdaten und/oder eine am besten bewertete Tätigkeit bei Einsatz von bestärkendem Lernen umfassen,
   - Bereitstellen der trainierten Unterstützungsfunktion (15).

2. Verfahren nach Anspruch 1, **dadurch gekennzeichnet, dass** der strukturierte Textdatensatz (11) der Trainingsdaten eine Log-Datei der wenigstens einen der wenigstens einen Eingriffseinrichtung (32, 35), die Bedienhandlungen und/oder eingestellte Betriebsparameter beschreibt, und/oder eine Konfigurationsdatei, die Bildaufnahmeparameter und/oder Eingriffsparameter für die Bildaufnahmeeinrichtung (33) enthält, und/oder ein Anteil einer solchen Konfigurationsdatei, insbesondere ein Metadatensatz, ist oder umfasst.

3. Verfahren nach Anspruch 1 oder 2, **dadurch gekennzeichnet, dass** als Eingangsdaten (14) für die Unterstützungsfunktion ausschließlich technisch von der wenigstens einen Eingriffseinrichtung (32, 35) verfügbare Datensätze und/oder Dateien verwendet werden und/oder die Eingangsdaten (14) multimodal sind.

4. Verfahren nach einem der vorangehenden Ansprüche, **dadurch gekennzeichnet, dass** bei dem Training und/oder im Rahmen einer zusätzlich bereitgestellten Überprüfungsfunktion zur Überprüfung der Ausgangsdaten wenigstens eine durch Ausgangsdaten beschriebene Tätigkeit mittels wenigstens einer Ausschlussbedingung (16) ausgeschlossen und/oder aus den Ausgangsdaten entfernt wird.

5. Verfahren nach Anspruch 4, **dadurch gekennzeichnet, dass** der Ausschluss für wenigstens eine der wenigstens einen Ausschlussbedingung (16) in Abhängigkeit eines Eingangsdatensatzes und/oder eines zugehörigen Ausgangsdatensatzes einer Anwendung der Unterstützungsfunktion und/oder für wenigstens eine der wenigstens einen Ausschlussbedingung (16) absolut erfolgt.

6. Verfahren nach einem der vorangehenden Ansprüche, **dadurch gekennzeichnet, dass** bei der Ermittlung von Ausgangsdaten, die eine auf den Eingriff selbst bezogene medizinische Tätigkeit beschreiben, in dem Trainingsvorgang zusätzlich zu wenigstens einem Teil der Eingangsdatensätze eine Experteninformation (9), die medizinisches Expertenwissen zu der durch den Eingangsdatensatz beschriebenen Eingriffssituation beschreibt, verwendet wird.

7. Verfahren nach Anspruch 6, **dadurch gekennzeichnet, dass** die Experteninformation (9) wenigstens teilweise eine von einem Experten vorgeschlagene und/oder bei der Aufzeichnung des Eingangsdatensatzes durchgeführte medizinische Solltätigkeit beschreibt, die als Grundwahrheit verwendet wird, und/oder im Rahmen des bestärkenden Lernens als Bewertung des einem jeweiligen Eingangsdatensatz zugehörigen Ausgangsdatensatzes bereitgestellt wird.

8. Verfahren nach Anspruch 6 oder 7, **dadurch gekennzeichnet, dass** die Experteninformation (9) wenigstens teilweise aus einer benutzerseitigen Annotation und/oder unter Verwendung einer mittels Expertenwissen vorab trainierten Expertisefunktion, die insbesondere wenigstens die Bilddaten eines jeweiligen Eingangsdatensatzes als Eingangsdaten nutzt, ermittelt wird.

9. Verfahren nach einem der Ansprüche 6 bis 8, **dadurch gekennzeichnet, dass** die Trainingsdatensätze (8) wenigstens teilweise auch eine das klinische Ergebnis des Eingriffs, insbesondere bezogen auf die Gesundheit des Patienten, beschreibende Ergebnisinformation (12) für jeweilige Eingriffe umfassen, wobei die Ausgangsdaten eine abgeschätzte und/oder referenzierte Prädiktionsinformation, die ein prädiziertes klinisches Ergebnis beschreibt, bei Durchführung der Tätigkeit umfassend ermittelt werden.

10. Verfahren nach einem der vorangehenden Ansprüche, **dadurch gekennzeichnet, dass** für Ausgangsdaten, die unter Nutzung eines Eingangsdatensatzes für einen ersten der Zeitpunkte bestimmt werden und als empfohlene Tätigkeit eine technische Bedienhandlung an wenigstens einer der wenigstens einen Eingriffseinrichtung (32, 35) beschreiben, das Training derart durchgeführt wird, dass die in dem zugehörigen strukturierten Textdatensatz (11), insbesondere einer Log-Datei, für den dem ersten Zeitpunkt folgenden Zeitpunkt beschriebene Bedienhandlung als Grundwahrheit verwendet wird.

11. Verfahren nach einem der vorangehenden Ansprüche, **dadurch gekennzeichnet, dass** die Unterstützungsfunktion so trainiert wird, dass die Ausgangsdaten Verweise auf Bild- und/oder Videomaterial, insbesondere der Trainingsdatensätze (8), das eine vergleichbare Eingriffssituation illustriert, umfassen.

12. Verfahren nach einem der vorangehenden Ansprüche, **dadurch gekennzeichnet, dass** wenigstens ein Teil der Trainingsdatensätze (8), der in einer letzten Trainingsphase zum Training verwendet wird, eine gemeinsame Eingriffsstrategie zur Durchführung des Eingriffs beschreibt, insbesondere eine in einer Arbeitsumgebung, in der die trainierte Unterstützungsfunktion (15) angewendet werden soll, einzusetzende Eingriffsstrategie.

13. Computerimplementiertes Verfahren zur Unterstützung einer Bedienperson bei einem bildüberwachten medizinischen Eingriff an einer Eingriffsanordnung (31), die wenigstens eine technische Eingriffseinrichtung (32, 35) aufweist, von der wenigstens eine eine medizinische Bildaufnahmeeinrichtung (33) zur Bildüberwachung des Eingriffs ist, umfassend folgende Schritte:

- zu einem Zeitpunkt während des Eingriffs, Zusammenstellen eines Eingangsdatensatzes, der den Ablauf des Eingriffs bis zu dem Zeitpunkt beschreibt und

  o wenigstens einen Bilddatensatz (10) der Bildüberwachung mit der Bildaufnahmeeinrichtung (33), der von der Bildaufnahmeeinrichtung (33) empfangen wird, und
  o wenigstens einen strukturierten Textdatensatz (11), der wenigstens eine durchgeführte technische Bedienhandlung und/oder wenigstens eine eingriffsbezogene Eingriffsinformation beschreibt und von wenigstens einer der wenigstens einen Eingriffseinrichtung (32, 35) empfangen wird, enthält,

- Anwenden einer trainierten Unterstützungsfunktion (15) auf den Eingangsdatensatz zur Ermittlung von Ausgangsdaten, die zumindest eine empfohlene, als nächstes durchzuführende Tätigkeit der Bedienperson beschreiben,
- Verwendung der Ausgangsdaten zur Unterstützung der Bedienperson.

14. Verfahren nach Anspruch 13, **dadurch gekennzeichnet, dass** die Ausgangsdaten auf eine mittels einer Benutzereingabe der Bedienperson ermittelte Anforderung ermittelt und an die Bedienperson ausgegeben werden.

15. Verfahren nach Anspruch 13 oder 14, **dadurch gekennzeichnet, dass**

  - die Ausgangsdaten wiederholt zu mehreren Zeitpunkten während des Eingriffs ermittelt werden,
  - eine Abweichung einer aktuellen, insbesondere noch nicht abgeschlossenen und/oder noch nicht wirkenden, Tätigkeit der Bedienperson von der gemäß den Ausgangsdaten empfohlenen Tätigkeit gemäß einer Metrik ermittelt wird, und
  - bei Überschreitung eines Schwellwerts für die Abweichung die Ausgangsdaten, insbesondere unter Bezug auf und/oder Vergleich mit der aktuellen Tätigkeit, an die Bedienperson als Empfehlung ausgegeben werden.

16. Verfahren nach Anspruch 15, **dadurch gekennzeichnet, dass** bei einer übereinstimmenden oder einer gleichen Klasse von Tätigkeiten angehörender aktueller Tätigkeit und empfohlener Tätigkeit die Metrik als eine Differenz einer Wirkungsstärke der jeweiligen Tätigkeiten ermittelt wird.

17. Bereitstellungseinrichtung (24) zum Bereitstellen einer trainierten Unterstützungsfunktion (15) für einen bildüberwachten medizinischen Eingriff an einer Eingriffsanordnung (31), die wenigstens eine technische Eingriffseinrichtung (32, 35) aufweist, von der wenigstens eine eine medizinische Bildaufnahmeeinrichtung (33) zur Bildüberwachung des Eingriffs ist, zur Unterstützung einer Bedienperson, umfassend:

- eine erste Bereitstellungsschnittstelle (26) zum Empfang einer für mehrere Datentypen, umfassend zwei und/oder dreidimensionale Bilder und Videos und strukturierten Text, vortrainierten generativen Funktion (1), die aus Eingangsdaten (3) der Datentypen auf die Eingangsdaten (3) bezogene Ausgangsdaten (2) generiert, als untrainierte Unterstützungsfunktion,
- eine zweite Bereitstellungsschnittstelle (27) zum Empfang von Trainingsdatensätzen (8), wobei die Trainingsdatensätze (8) für mehrere Zeitpunkte einer Eingriffsdauer jedes in den Trainingsdatensätzen (8) beschriebenen Eingriffs wenigstens folgende Eingangsdaten (14) für die Unterstützungsfunktion, die den Ablauf des Eingriffs bis zu dem jeweiligen Zeitpunkt beschreiben, enthalten:

o wenigstens einen Bilddatensatz (10) der Bildüberwachung mit der Bildaufnahmeeinrichtung (33) und
o wenigstens einen strukturierten Textdatensatz (11), der wenigstens eine durchgeführte technische Bedienhandlung und/oder wenigstens eine eingriffsbezogene Eingriffsinformation beschreibt und seitens wenigstens einer der wenigstens einen Eingriffseinrichtung (32, 35) vorliegt,

- eine Trainingseinheit (28) zum Trainieren der Unterstützungsfunktion mit den Trainingsdatensätzen (8), wobei das Training derart erfolgt, dass bei Trainingsdaten bis zu einem Zeitpunkt enthaltenden Eingangsdaten (14) die Ausgangsdaten zumindest eine empfohlene Tätigkeit für die Bedienperson beschreiben und die empfohlene Tätigkeit die nächstfolgende Tätigkeit der Bedienperson gemäß den Trainingsdaten und/oder eine am besten bewertete Tätigkeit bei Einsatz von bestärkendem Lernen umfasst, und
- eine dritte Bereitstellungsschnittstelle (30) zum Bereitstellen der trainierten Unterstützungsfunktion (15).

18. Computerprogramm, welches, wenn es auf einer Bereitstellungseinrichtung (24) ausgeführt wird, diese veranlasst, die Schritte eines Verfahrens nach einem der Ansprüche 1 bis 12 durchzuführen.

19. Elektronisch lesbarer Datenträger, auf dem ein Computerprogramm nach Anspruch 18 gespeichert ist.

20. Steuereinrichtung (37) zur Unterstützung einer Bedienperson bei einem bildüberwachten medizinischen Eingriff an einer Eingriffsanordnung (31), die die Steuereinrichtung (37) und wenigstens eine technische Eingriffseinrichtung (32, 35) aufweist, von der wenigstens eine eine medizinische Bildaufnahmeeinrichtung (33) zur Bildüberwachung des Eingriffs ist, umfassend:

- eine erste Anwendungsschnittstelle (40) zum Empfang von wenigstens einem Bilddatensatz (10) der Bildüberwachung von der Bildaufnahmeeinrichtung (33) und wenigstens einem strukturierten Textdatensatz (11), der wenigstens eine durchgeführte technische Bedienhandlung und/oder wenigstens eine eingriffsbezogene Eingriffsinformation beschreibt, von wenigstens einer der wenigstens einen Eingriffseinrichtung (32, 35),
- eine Zusammenstellungseinheit (41) zum Zusammenstellen eines Eingangsdatensatzes zu einem Zeitpunkt während des Eingriffs, wobei der Eingangsdatensatz den Ablauf des Eingriffs bis zu dem Zeitpunkt beschreibt und den wenigstens einen Bilddatensatz (10) und den wenigstens einen strukturierten Textdatensatz (11) enthält,
- eine Anwendungseinheit (42) zum Anwenden einer trainierten Unterstützungsfunktion (15) auf den Eingangsdatensatz zur Ermittlung von Ausgangsdaten, die zumindest die zumindest eine empfohlene, als nächstes durchzuführende Tätigkeit der Bedienperson beschreiben, und
- eine Unterstützungseinheit (45) zur Verwendung der Ausgangsdaten zur Unterstützung der Bedienperson.

21. Eingriffsanordnung (31), aufweisend eine Steuereinrichtung (37) nach Anspruch 20 und wenigstens eine technische Eingriffseinrichtung (32, 35), von der wenigstens eine eine medizinische Bildaufnahmeeinrichtung (33) zur Bildüberwachung des Eingriffs ist.

22. Computerprogramm, welches, wenn es auf einer Steuereinrichtung (37) einer Eingriffsanordnung (31) ausgeführt wird, diese veranlasst, die Schritte eines Verfahrens nach einem der Ansprüche 13 bis 16 durchzuführen.

23. Elektronisch lesbarer Datenträger, auf dem ein Computerprogramm nach Anspruch 22 gespeichert ist.

FIG 1

FIG 2

## FIG 3

## FIG 4

FIG 5

FIG 6

FIG 7

**EUROPÄISCHER RECHERCHENBERICHT**

Europäisches Patentamt
European Patent Office
Office européen des brevets

Nummer der Anmeldung

EP 24 18 6811

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2023/317227 A1 (SINHA AYUSHI [US] ET AL) 5. Oktober 2023 (2023-10-05) | 1-10, 12-23 | INV.<br>G16H20/40 |
| Y | * Das gesamte Dokument, insbesondere: Paragraphen[0004], [0005], [0014], [0026], [0027], [0030], [0034], [0036] - [0050], [0053] - [0055], [0060] - [0062], [0079]; Abbildungen 1, 2. *<br>----- | 11 | G16H10/60<br>A61B34/00<br>G06F18/00<br>G06N5/00<br>G06N20/00<br>G16H30/40 |
| X | WO 2021/211603 A1 (KALIBER LABS INC [US]) 21. Oktober 2021 (2021-10-21) | 1-10, 12-23 | G16H50/70<br>G16H50/30 |
| Y | * Das gesamte Dokument, insbesondere: Paragraphen [0006], [0015], [0077], [0113] - [0121]; Abbildungen 9, 10, 12; Ansprüche 1, 2, 24, 28, 29, 30, 35, 38. *<br>----- | 11 | ADD.<br>G16H70/00 |
| X | WO 2020/056086 A1 (ORTHOGRID SYSTEMS INC [US]; ORTHOGRID SYSTEMS SAS [FR]) 19. März 2020 (2020-03-19) | 1-10, 12-23 | |
| Y | * Das gesamte Dokument, insbesondere: Paragraphen [0006] - [0011], [0086], [0087], [0093], [0094], [0099], [0109] - [0117], [0170], [0174] - [0176], [0180]; Abbildungen 1A, 1B, 2B, 3B, 4B, 5B, 5C; Ansprüche 1 - 3, 11. *<br>----- | 11 | |
| X | EP 4 231 310 A1 (LEICA INSTR SINGAPORE PTE LTD [SG]) 23. August 2023 (2023-08-23) | 1-10, 12-23 | |
| Y | * Das gesamte Dokument, insbesondere: Paragraphen [0002] - [0020], [0024], [0080], [0081], [0100], [0102]; Ansprüche 1, 3, 5, 6. *<br>----- | 11 | |
| Y | WO 2019/226182 A1 (VERB SURGICAL INC [US]) 28. November 2019 (2019-11-28) | 11 | |
| A | * Das gesamte Dokument, insbesondere: Paragraphen [0034], [0065]; Abbildungen 1, 3 - 5. *<br>----- | 1-10, 12-23 | |

| RECHERCHIERTE SACHGEBIETE (IPC) |
|---|
| G16H<br>G06N<br>G06V<br>A61B<br>G06F |

Der vorliegende Recherchenbericht wurde für alle Patentansprüche erstellt

| Recherchenort | Abschlußdatum der Recherche | Prüfer |
|---|---|---|
| München | 17. Dezember 2024 | Ruschke, Stefan |

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**  EP 24 18 6811

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

17-12-2024

| Im Recherchenbericht angeführtes Patentdokument | Datum der Veröffentlichung | Mitglied(er) der Patentfamilie | | Datum der Veröffentlichung |
|---|---|---|---|---|
| US 2023317227 A1 | 05-10-2023 | KEINE | | |
| WO 2021211603 A1 | 21-10-2021 | EP | 4135566 A1 | 22-02-2023 |
| | | JP | 2023523560 A | 06-06-2023 |
| | | US | 2023245753 A1 | 03-08-2023 |
| | | WO | 2021211603 A1 | 21-10-2021 |
| WO 2020056086 A1 | 19-03-2020 | EP | 3852645 A1 | 28-07-2021 |
| | | JP | 7466928 B2 | 15-04-2024 |
| | | JP | 2022500148 A | 04-01-2022 |
| | | JP | 2024099519 A | 25-07-2024 |
| | | US | 2021015560 A1 | 21-01-2021 |
| | | US | 2021177522 A1 | 17-06-2021 |
| | | US | 2023157765 A1 | 25-05-2023 |
| | | WO | 2020056086 A1 | 19-03-2020 |
| EP 4231310 A1 | 23-08-2023 | CN | 119013733 A | 22-11-2024 |
| | | EP | 4231310 A1 | 23-08-2023 |
| | | WO | 2023156406 A1 | 24-08-2023 |
| WO 2019226182 A1 | 28-11-2019 | CN | 110996748 A | 10-04-2020 |
| | | EP | 3796827 A1 | 31-03-2021 |
| | | JP | 7074893 B2 | 24-05-2022 |
| | | JP | 2021528724 A | 21-10-2021 |
| | | US | 2019362834 A1 | 28-11-2019 |
| | | US | 2022148702 A1 | 12-05-2022 |
| | | US | 2024242818 A1 | 18-07-2024 |
| | | WO | 2019226182 A1 | 28-11-2019 |

EPO FORM P0461

Für nähere Einzelheiten zu diesem Anhang : siehe Amtsblatt des Europäischen Patentamts, Nr.12/82